# EUROPÄISCHE PATENTSCHRIFT

(11) **EP 4 288 400 B1**
(45) Veröffentlichungstag und Bekanntmachung des Hinweises auf die Patenterteilung: **11.12.2024**
(21) Anmeldenummer: 22702932.9
(22) Anmeldetag: 27.01.2022
(51) Int. Cl.: C07C 29/70, C07C 29/80, C07C 31/30

(54) **VERFAHREN ZUR ENERGIEEFFIZIENTEN HERSTELLUNG VON ALKALIMETALLALKOHOLATEN**
METHOD FOR THE ENERGY-EFFICIENT PRODUCTION OF ALKALI METAL ETHANOLATES
PROCÉDÉ DE PRODUCTION ÉCONOMIQUE EN ÉNERGIE D'ALCOOLATES ALCALIMÉTALES

(30) Priorität: 05.02.2021 EP 21155473
(43) Veröffentlichungstag der Anmeldung: 13.12.2023
(73) Patentinhaber: Evonik Operations GmbH, 45128 Essen (DE)
(72) Erfinder: ROETTGER, Dirk, 50672 Köln (DE); REIMANN, Sebastian, 50389 Wesseling (DE); PAUL, Niklas, 45770 Marl (DE); RIX, Armin Matthias, 45770 Marl (DE); ZITZEWITZ, Philip, 45721 Haltern am See (DE); SCHRÖDER, Moritz, 48153 Münster (DE)
(74) Vertreter: Evonik Patent Association
(86) Internationale Anmeldenummer: PCT/EP2022/051869
(87) Internationale Veröffentlichungsnummer: WO 2022/167311

(56) Entgegenhaltungen:
- EP-A1- 1 997 794
- WO-A1-01/42178
- WO-A1-2010/097318

## Beschreibung

Die vorliegende Erfindung betrifft ein Verfahren zur Herstellung von Alkalimetallalkoholaten im Gegenstrom durch Reaktivrektifikation, wobei das Alkalimetall aus Natrium und Kalium ausgewählt ist. Das Verfahren erfolgt in mindestens einer Reaktionskolonne und mindestens einer Rektifikationskolonne. Das erfindungsgemäße Verfahren zeichnet sich dadurch aus, dass der zum Betrieb der Kolonnen und somit zur Durchführung des Verfahrens genutzte Heizdampf kondensiert, und die Energie im erhaltenen Kondensat eingesetzt wird.

### 1. Hintergrund der Erfindung

Die Herstellung von Alkalimetallalkoholaten ist ein wichtiger industrieller Prozess.

Alkalimetallalkoholate werden als starke Basen in der Synthese zahlreicher Chemikalien, z.B. bei der Herstellung von Pharma- oder Agrowirkstoffen, eingesetzt. Weiterhin finden Alkalimetallalkoholate Anwendung als Katalysatoren in Umesterungs- und Amidierungsreaktionen.

Alkalimetallalkoholate (MOR) werden mittels Reaktivdestillation in einer Gegenstromdestillationskolonne aus Alkalimetallhydroxiden (MOH) und Alkoholen (ROH) hergestellt, wobei das gemäß folgender Reaktion <1> entstehende Reaktionswasser mit dem Destillat entfernt wird.

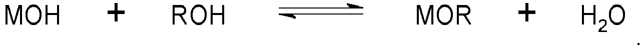

Ein solches Verfahrensprinzip ist beispielsweise in der US 2,877,274 A beschrieben, wobei wässrige Alkalimetallhydroxid-Lösung und gasförmiges Methanol im Gegenstrom in einer Rektifikationskolonne gefahren werden. In prinzipiell nicht veränderter Form wird dieses Verfahren in der WO 01/42178 A1 erneut beschrieben.

Ähnliche Verfahren, bei denen jedoch zusätzlich noch ein Schleppmittel, wie beispielsweise Benzol, eingesetzt wird, sind in der GB 377,631 A und der US 1,910,331 A beschrieben. Das Schleppmittel dient hierbei zur Trennung von Wasser und dem wasserlöslichen Alkohol, und das Kondensat wird einer Phasentrennung unterzogen, um das Reaktionswasser abzutrennen.

Entsprechend beschreibt die DE 96 89 03 C ein Verfahren zur kontinuierlichen Herstellung von Alkalimetallalkoholaten in einer Reaktionskolonne, wobei das am Kopf entnommene Wasser-Alkohol-Gemisch kondensiert und anschließend einer Phasentrennung unterworfen wird. Die wässrige Phase wird hierbei verworfen und die alkoholische Phase wird zusammen mit dem frischen Alkohol der Kolonne am Kopf zurückgegeben. Ein ähnliches Verfahren beschreibt die EP 0 299 577 A2, wobei die Wasserabtrennung im Kondensat mit Hilfe einer Membran erfolgt.

Die industriell wichtigsten Alkalimetallalkoholate sind jene des Natriums und Kaliums, und hierbei insbesondere die Methylate und Ethylate. Deren Synthese ist vielfach im Stand der Technik beschrieben, zum Beispiel in der EP 1 997 794 A1.

Bei den im Stand der Technik beschriebenen Synthesen der Alkalimetallalkoholate durch Reaktivrektifikation werden üblicherweise Brüden erhalten, welche den eingesetzten Alkohol und Wasser umfassen. Es ist aus wirtschaftlichen Gründen sinnvoll, den von den Brüden umfassten Alkohol wieder in der Reaktivdestillation als Edukt einzusetzen. Deshalb werden die Brüden üblicherweise einer Rektifikationskolonne zugeführt und der darin enthaltene Alkohol abgetrennt (beschrieben beispielsweise in der GB 737 453 A und der US 4,566,947 A). Der so zurückgewonnene Alkohol wird dann beispielsweise als Edukt der Reaktivdestillation zugeführt. Alternativ oder zusätzlich kann ein Teil des Alkoholbrüden zur Beheizung der Rektifikationskolonne genutzt werden (beschrieben in WO 2010/097318 A1). Dazu muss der Brüden allerdings verdichtet werden, um das zur Beheizung der Rektifikationskolonne nötige Temperaturniveau zu erreichen.

Die zum Betrieb des Verfahrens nötige Energie wird normalerweise über Heizdampf in das erfindungsgemäße Verfahren eingetragen, zum Beispiel über Wärmeübertrager (alternativer Begriff "Wärmetauscher") wie zum Beispiel Verdampfer am Sumpf der Kolonne oder zur Beheizung der Alkohol- oder Alkalimetallhydroxidlösung. Bei der Übertragung der Energie kondensiert der Heizdampf, und die erhaltene flüssige Phase, das Heizdampfkondensat, enthält häufig noch beträchtliche Mengen an Restwärme. Daraus ergeben sich folgende Nachteile: Einerseits ist es energetisch unerwünscht, diese Energie ungenutzt dissipieren zu lassen. Daneben werden im industriellen Kontext (Chemieparks und Technologieparks) Kondensate üblicherweise in einem Kondensatnetz gesammelt. Wenn sich dieses aus verschiedenen Anlagen speist, unterscheiden sich die zugeführten Kondensate in Temperatur und Druck. Um Druckschläge im Kondensatnetz zu verhindern, muss jede an das Netz angeschlossene Anlage deshalb das entsprechende Kondensat aktiv kühlen, was wiederum zusätzlichen Energieaufwand erfordert.

Die Aufgabe der vorliegenden Erfindung bestand demnach darin, ein Verfahren zur Herstellung von Alkalimetallalkoholaten zur Verfügung zu stellen, welches die vorgenannten Nachteile behebt und insbesondere die effiziente Nutzung der Energie in den Heizdampfkondensaten erlaubt. Neben der vorteilhaften Energieeinsparung soll dies auch die leichte, energieeffiziente Einstellung des Kondensats auf bestimmte Druck- und Temperaturwerte erlauben.

Es wurde nun überraschenderweise ein Verfahren gefunden, welches diese Aufgabe löst.

### 2. Kurzzusammenfassung der Erfindung

Die vorliegende Erfindung betrifft demnach ein Verfahren gemäß Anspruch 1.

### 3. Abbildungen

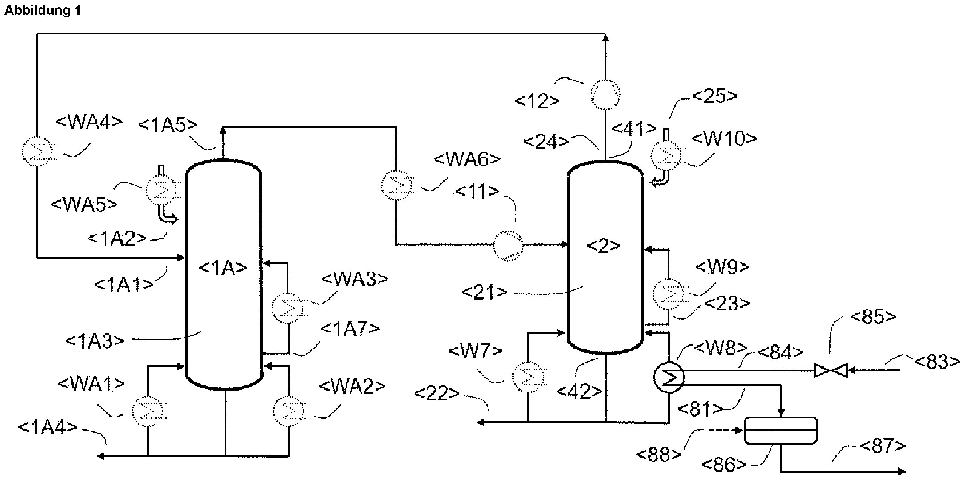

**Abbildung 1** zeigt ein nicht erfindungsgemäßes Verfahren zur Herstellung von Alkalimetallalkoholaten mit verschiedenen Wärmeübertragern.

Eine solche Anlage umfasst eine Reaktionskolonne **RR_{A}** <1A>. Am Kopf der Reaktionskolonne **RR_{A}** <1A> wird eine wässrige NaOH-Lösung als Eduktstrom **S_{AE2}** <1A2> zugegeben. Alternativ kann z.B. auch eine methanolische NaOH- oder KOH-Lösung als Eduktstrom **S_{AE2}** <1A2> zugegeben werden, um dann das entsprechende Natrium- bzw. Kaliummethylat herzustellen. Dem Eduktstrom **S_{AE2}** <1A2> kann gegebenenfalls über einen Wärmeübertrager **WT** <WA5> Energie zugeführt werden, z.B. um diesen vorzuwärmen. Oberhalb des Sumpfes der Reaktionskolonne **RR_{A}** <1A> wird Methanol als Eduktstrom **S_{AE1}** <1A1> dampfförmig zugegeben. Dem Eduktstrom **S_{AE1}** <1A1> kann gegebenenfalls über einen Wärmeübertrager **WT** <WA4> Energie zugeführt werden, z.B. um diesen vorzuwärmen. Durch die Umsetzung der beiden Eduktströme **S_{AE1}** <1A1> und **S_{AE2}** <1A2> wird in der Reaktionskolonne **RR_{A}** <1A> ein Rohproduktgemisch **RP_{A}** <1A3> erhalten, welches NaOCH₃, Wasser, CH₃OH, NaOH umfasst, wenn **S_{AE2}** <1A2> NaOH umfasst. Wenn **S_{AE2}** <1A2> KOH umfasst, wird ein Rohproduktgemisch **RP_{A}** <1A3> erhalten, welches KOCH₃, Wasser, CH₃OH und KOH umfasst.

Am Sumpf der Reaktionskolonne **RR_{A}** <1A> wird der Produktstrom **S_{AS}** <1A4>, der Natriummethanolat bzw. Kaliummethanolat gelöst in Methanol umfasst, erhalten. Mit dem optionalen Sumpfverdampfer **VS_{RRA}** <WA1> am Sumpf der Kolonne **RR_{A}** <1A> kann die Konzentration der Natrium- bzw. Kaliummethylatlösung **S_{AS}** <1A4> auf den gewünschten Wert eingestellt werden. Durch den optionalen Sumpfverdampfer **VS_{RRA}** <WA2> kann gegebenenfalls Energie auf einen Teil von **S_{AS}** <1A4> übertragen werden, der in die Reaktionskolonne **RR_{A}** <1A> rezykliert wird. **VS_{RRA}** <WA2> dient dabei insbesondere auch zum Anfahren der Kolonne **RR_{A}** <1A>. Der optionale Zwischenverdampfer **VZ_{RRA}** <WA3> eröffnet eine Möglichkeit, Energie auf das Rohproduktgemisch **RP_{A}** <1A3> zu übertragen. Dieses wird der Kolonne **RR_{A}** <1A> als Strom **S_{RRAZ}** <1A7> entnommen, der Strom **S_{RRAZ}** <1A7> im Zwischenverdampfer **VZ_{RRA}** <WA3> erwärmt und dann in die Kolonne **RR_{A}** <1A> zurückgeführt.

Am Kopf der Reaktionskolonne **RR_{A}** <1A> wird ein Brüdenstrom **S_{AB}** <1A5> entnommen. Ein Teil des Brüdenstroms **S_{AB}** <1A5> kann am Kopf der Reaktionskolonne **RR_{A}** <1A> in einem Kondensator **K_{RRA}** kondensiert und flüssig als Rücklauf auf den Kopf der Reaktionskolonne **RR_{A}** <1A> aufgegeben werden. Diese nicht in Abbildung 1 gezeigte Ausführungsform, d.h. die Einstellung des Rücklaufs am Kopf der Reaktionskolonne **RR_{A}** <1A>, ist jedoch optional.

Der Brüdenstrom **S_{AB}** <1A5> wird, optional über einen Wärmeübertrager **WT** <WA6>, über einen Verdichter **VD₃₁** <11>, einer Rektifikationskolonne **RD_{A}** <2> zugeleitet. In der Rektifikationskolonne **RD_{A}** <2> wird ein Gemisch **G_{A}** <21> erhalten, welches Wasser und Methanol umfasst, welches in **RD_{A}** <2> in einen Brüdenstrom **S_{DAB}** <24> umfassend Methanol und einen Sumpfstrom **S_{DAS}** <22> umfassend Wasser und ROH getrennt wird. Am Sumpf der Kolonne **RD_{A}** <2> wird der Sumpfstrom **S_{DAS}** <22> an einer Entnahmestelle **E_{AK}** <42> abgeführt und kann teilweise wieder über den optionalen Sumpfverdampfer **VS_{A}** <W7> oder den Sumpfverdampfer **VS_{A}** <W8> in die Kolonne **RD_{A}** <2> rückgeführt werden.

Am Kopf der Rektifikationskolonne **RD_{A}** <2> wird Methanolbrüden **S_{DAB}** <24> an einer Entnahmestelle **E_{AK}** <41> abgeführt. Dieser wird dann optional über den Verdichter <12> geleitet und zur Reaktionskolonne **RR_{A}** <1A> rezykliert, wo er als Eduktstrom **S_{AE1}** <1A1> eingesetzt wird. Der Verdichter <12> kann auch anstatt des Verdichters **VD₃₁** <11> eingesetzt werden.

Der Zwischenverdampfer **VZ_{A}** <W9> eröffnet eine Möglichkeit, Energie auf das Gemisch **G_{A}** <21> zu übertragen. Dieses wird der Kolonne **RD_{A}** <2> als Strom **S_{AZ}** <23> entnommen, der Strom **S_{AZ}** <23> im Zwischenverdampfer **VZ_{A}** <W9> erwärmt und dann in die Kolonne **RD_{A}** <2> zurückgeführt.

Ein Strom von Frischmethanol <25> kann dem System über die Rektifikationskolonne **RD_{A}** <2> zugeführt werden. Diesem kann über einen Wärmeübertrager **WT** <W10> Energie zugeführt werden. Das Frischmethanol kann dabei der Rektifikationskolonne **RD_{A}** <2> direkt zugegeben werden.

Durch den Sumpfverdampfer **VS_{A}** <W8> wird Energie auf einen Teil von **S_{DAS}** <22> übertragen werden, der in die Rektifikationskolonne **RD_{A}** <1A> rezykliert wird. Der Sumpfverdampfer **VS_{A}** <W8> dient dabei insbesondere auch zum Anfahren der Kolonne **RD_{A}** <2>. Abbildung 1 zeigt anhand des Sumpfverdampfers **VS_{A}** <W8> die Beheizung mit Heizdampf H₁ <84> anhand des Standes der Technik. Demnach wird über den Sumpfverdampfer **VS_{A}** <W8> Energie von Heizdampf **H₁** <84> auf rezykliertes **S_{DAS}** <22> übertragen und somit letztendlich für die destillative Trennung des Gemischs **G_{A}** <21> zur Verfügung gestellt.

Die Kondensationstemperatur des Heizdampfs **H₁** <84> muss je nach benötigter Heizleistung der Destillation eingestellt werden. Da die Temperatur bzw. der Druck des in industriellen Anlagen zur Verfügung stehenden Heizdampfs **H₃** <83> üblicherweise von den im konkreten Fall gewünschten Werten abweicht, muss **H₃** <83> zunächst zu **H₁** <84> entspannt werden, was über ein Regelungsventil <85> erfolgt. Dies erfolgt unter Dissipation von Teilen der im Heizdampf **H₃** <83> enthaltenen Energie.

Durch die Übertragung von Energie von **H₁** <84> auf **S_{DAS}** <22> im Sumpfverdampfer **VS_{A}** <W8> kondensiert **H₁** <84> mindestens teilweise zum Kondensat **K₁** <81>, welches in einem Kondensatbehälter <86> gesammelt wird, wo gegebenenfalls Kondensaten <88> aus anderen Wärmeübertragern der Anlage nach Abbildung 1 oder sogar anderen Anlagen (z.B. bei Verbundstandorten) gesammelt werden. Die gesammelten Kondensate müssen dann als Kondensatstrom <87> entsorgt werden oder können etwa in Chemieparks ins Kondensatnetz eingespeist werden. Damit wird nicht nur ein Teil der in den Kondensaten vorhandenen Energie ungenutzt verworfen, es ist in vielen Fällen auch nötig, diese unter zusätzlichem Energieaufwand auf eine bestimmte Temperatur einzustellen, um Druckschläge im Kondensatnetz zu vermeiden.

Diese Problematik stellt sich nicht nur in dem Fall, in dem des Heizdampf **H₁** <84> den Sumpfverdampfer **VS_{A}** <W8> beheizt. Bei Anlagen, die jener in Abbildung 1 entsprechen, in denen aber anstatt über oder zusätzlich zu dem Sumpfverdampfer **VS_{A}** <W8> über einen der anderen Wärmetauscher Energie von Heizdampf **H₃** <83> eingetragen werden soll, stellt sich dasselbe Problem.

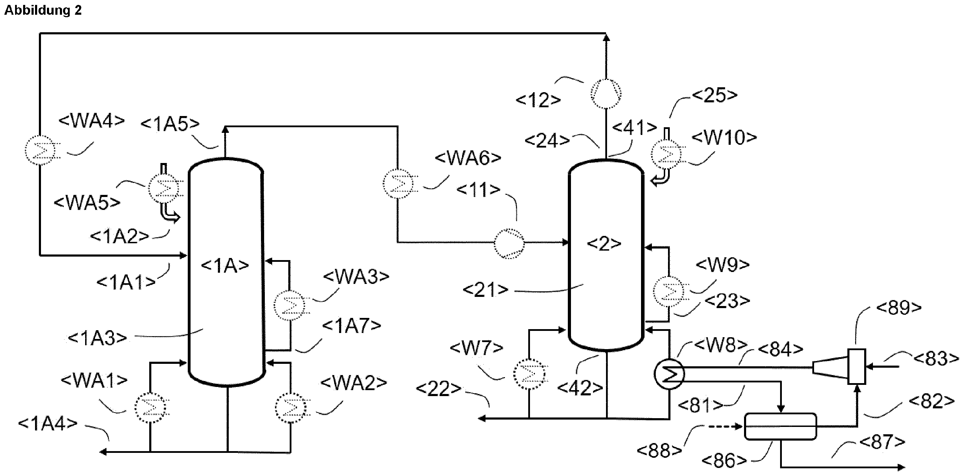

**Abbildung 2** zeigt eine Ausführungsform des erfindungsgemäßen Verfahrens. Die Anlage entspricht der in Abbildung 1 beschriebenen. Das charakteristische Merkmal des erfindungsgemäßen Verfahrens wird anhand des Sumpfverdampfers **VS_{A}** <W8> erläutert. Die entsprechende Führung des Heizdampfes **H₁** ist aber alternativ oder zusätzlich auch an einem der anderen Wärmeübertrager **WT** <WA1> bis <WA6>, <W7>, <W9>, <W10> möglich.

Entsprechend dem Vorgehen im Stand der Technik wird auch hier über den Sumpfverdampfer **VS_{A}** <W8> Energie von Heizdampf **H₁** <84> auf **S_{DAS}** <22> übertragen und somit letztendlich für die destillative Trennung des Gemischs **G_{A}** <21> zur Verfügung gestellt. Dadurch wird ein Heizdampfkondensat **K₁** <81> erhalten, welches im Kondensatbehälter <86>, gegebenenfalls mit anderen Kondensaten <88>, gesammelt und entspannt wird. Die Kondensate <88> können sich aus der Beheizung über einen der anderen Wärmeübertrager **WT** <WA1> bis <WA6>, <W7>, <W9>, <W10>, wenn die Anlage einen solchen aufweist, ergeben, oder, wenn das erfindungsgemäße Verfahren in der Nähe zu einer anderen Anlage durchgeführt wird, in der Heizdampfkondensat anfällt, von einer solchen Anlagen in den Kondensatbehälter <86> geleitet werden. Der entscheidende Unterschied zu den Verfahren des Standes der Technik besteht darin, dass mindestens ein Teil des Heizdampfkondensats **K₁** <81> entspannt wird, und dadurch ein Heizdampf **H₂** <82>, dessen Druck **p₂** geringer als der Druck **p₁** von **H₁** <84> ist, erhalten wird. Der dadurch erhaltene Heizdampf **H₂** <82> wird dann mit frischem Heizdampf **H₃** <83>, dessen Druck **p₃** > **p₂** und in der Ausführungsform gemäß Abbildung 2 auch **p₃** > **p₁** ist, vermischt. Dadurch wird der Heizdampf/Kondensat/Heizdampf-Kreislauf aus **H₁** <84> / **K₁** <81> / **H₂** <82> vervollständigt, und es wird neuer Heizdampf **H₄** erhalten, der gleich ist wie der Heizdampf **H₁** <84> mit gewünschtem Druck (und somit gewünschter Temperatur) und in der neuen Runde als Heizdampf **H₁** <84> wieder zur Beheizung des Sumpfverdampfers **VS_{A}** <W8> eingesetzt werden kann.

Im Gegensatz zu den Verfahren des Standes der Technik ergeben sich dadurch die folgenden Vorteile:
- Ein höherer Anteil der Energie im Kondensat **K₁** wird in das Verfahren integriert.
- Dadurch, dass die Energie im Kondensat **K₁** genutzt wird, muss das Kondensat **K₁** weniger gekühlt werden, bevor es ins Kondensatnetz eingespeist wird, um die Wahrscheinlichkeit von Druckschlägen im Kondensatnetz zu verringern. Dadurch wird zusätzlich Kühlenergie eingespart.

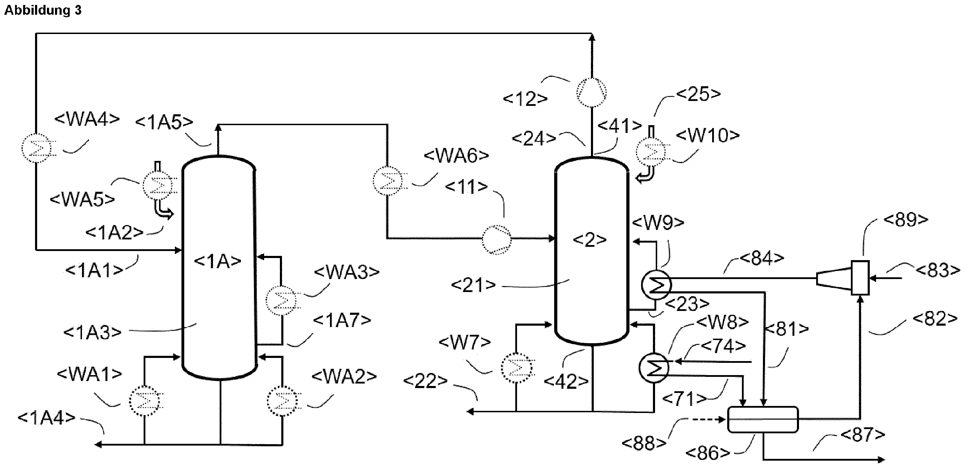

**Abbildung 3** zeigt eine weitere Ausführungsform des erfindungsgemäßen Verfahrens. Diese entspricht der in Abbildung 2 beschriebenen Ausführungsform bis darauf, dass an der Rektifikationskolonne **RD_{A}** <2> im Zwischenverdampfer **VZ_{A}** <W9> Energie von **H₁** <84> auf das Gemisch **G_{A}** <21> übertragen wird. Außerdem wird der Kondensatbehälter <86> auch mit Heizdampfkondensat <71> gespeist, welches durch Kondensation von Heizdampf <74> beim Betrieb des Sumpfverdampfers **VS_{A}** <W8> erhalten wird.

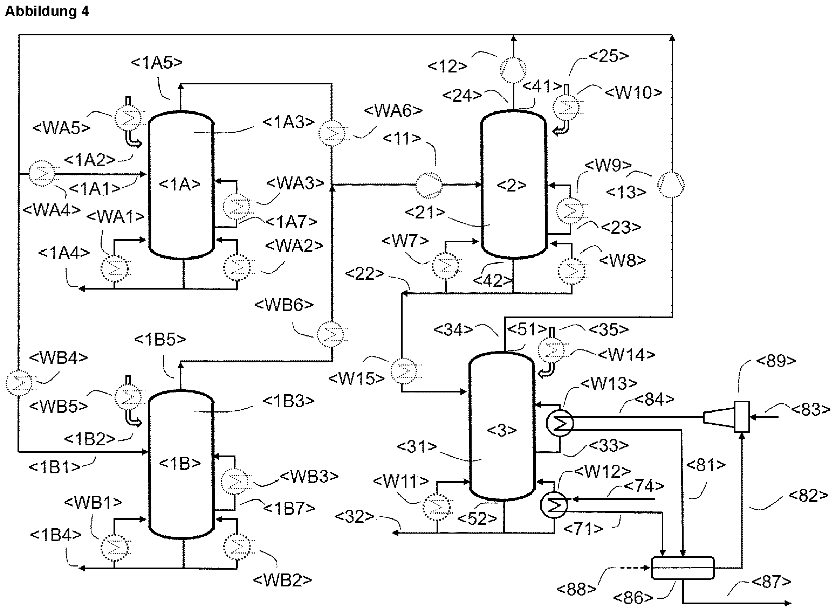

**Abbildung 4** zeigt eine weitere Ausführungsform des erfindungsgemäßen Verfahrens. Diese weist die in Abbildung 3 beschriebene Reaktionskolonne **RR_{A}** <1A> sowie die Rektifikationskolonne **RD_{A}** <2> auf. Zusätzlich weist die Ausführungsform gemäß Abbildung 4 die folgenden Merkmale auf:
Neben der Reaktionskolonne **RR_{A}** <1A> umfasst die im Verfahren verwendete Anlage eine weitere Reaktionskolonne **RR_{B}** <1B>, in welcher als Eduktstrom **S_{BE2}** <1B2> eine wässrige KOH-Lösung zugegeben wird. Es kann sich auch um die wässrige oder alkoholische Lösung von NaOH handeln, vorteilhafterweise wird **S_{BE2}** <1B2> jedoch so gewählt, dass das von diesem Strom umfasste Alkalimetall sich von dem in **S_{AE2}** <1A2> unterscheidet. Dem Eduktstrom **S_{BE2}**<1B2> kann gegebenenfalls über einen Wärmeübertrager **WT** <WB5> Energie zugeführt werden, z.B. um diesen vorzuwärmen. Wenn **S_{BE2}** <1B2> NaOH umfasst, wird **S_{BE2}** <1B2> in **RR_{B}** <1B> mit einem gegebenenfalls über den Wärmeübertrager WT <WB4> vorgewärmten Strom **S_{BE1}** <1B1 > umfassend CH₃OH zu einem Rohproduktgemisch **RP_{B}** <1B3> umfassend NaOCH₃, Wasser, CH₃OH, NaOH umgesetzt. Wenn **S_{BE2}** <1B2> KOH umfasst, wird ein Rohproduktgemisch **RP_{B}** <1B3> erhalten, welches KOCH₃, Wasser, CH₃OH und KOH umfasst. In dieser Ausführungsform ist ROH somit Methanol.

Am Sumpf der Reaktionskolonne **RR_{B}** <1B> wird der Produktstrom **S_{BS}** <1B4>, der Natriummethanolat bzw. Kaliummethanolat gelöst in Methanol umfasst, erhalten. Mit dem optionalen Sumpfverdampfer **VS_{RRB}** <WB1> am Sumpf der Kolonne **RR_{B}** <1 B> kann die Konzentration der Natrium- bzw. Kaliummethylatlösung **S_{BS}** <1A4> auf den gewünschten Wert eingestellt werden. Durch den optionalen Sumpfverdampfer **VS_{RRB}** <WB2> kann gegebenenfalls Energie auf einen Teil von **S_{BS}** <1B4> übertragen werden, der in die Reaktionskolonne **RR_{B}** <1B> rezykliert wird. **VS_{RRB}** <WB2> dient dabei insbesondere auch zum Anfahren der Kolonne **RR_{B}** <1B>. Der optionale Zwischenverdampfer **VZ_{RRB}** <WB3> eröffnet eine Möglichkeit, Energie auf das Rohproduktgemisch **RP_{B}** <1B3> zu übertragen. Dieses wird der Kolonne **RR_{B}** <1B> als Strom **S_{RRBZ}** <1B7> entnommen, letzterer im optionale Zwischenverdampfer **VZ_{RRB}** <WB3> erwärmt und dann in die Kolonne **RR_{B}** <1B> zurückgeführt.

Am Kopf der Reaktionskolonne **RR_{B}** <1B> wird ein Brüdenstrom **S_{BB}** <1B5> entnommen. Ein Teil des Brüdenstroms **S_{AB}** <1B5> kann am Kopf der Reaktionskolonne **RR_{B}** <1 B> in einem Kondensator kondensiert und flüssig als Rücklauf auf den Kopf der Reaktionskolonne **RR_{B}** <1 B> aufgegeben werden. Diese nicht in Abbildung 4 gezeigte Ausführungsform, d.h. die Einstellung des Rücklaufs am Kopf der Reaktionskolonne **RR_{B}** <1B>, ist jedoch optional.

Der Brüdenstrom **S_{BB}** <1B5> wird optional über einen Wärmeübertrager **WT** <WB6> geleitet. **S_{BB}** <1B5> wird mit dem Strom **S_{AB}** <1A5> vereinigt und dann, nachdem der vereinigte Strom über einen Verdichter **VD₃₁** <11> geführt wurde, einer Rektifikationskolonne **RD_{A}** <2> zugeleitet. Die beiden Ströme **S_{AB}** <1A5> und **S_{BB}** <1B5> können auch getrennt in die Rektifikationskolonne **RD_{A}** <2> geleitet werden und sich erst in dieser vereinigen.

In der Rektifikationskolonne **RD_{A}** <2> findet die destillative Trennung des Gemisches **G_{A}** <21> in einen Brüdenstrom **S_{DAB}** <24> umfassend ROH und einen Sumpfstrom **S_{DAS}** <22> umfassend Wasser und ROH statt. Der Sumpfstrom **S_{DAS}** <22> wird an einer Entnahmestelle **E_{AK}** <42> am Sumpf der Kolonne **RD_{A}** <2> abgeführt und kann teilweise über den optionalen Sumpfverdampfer **VS_{A}** <W7> oder den Sumpfverdampfer **VS_{A}** <W8>am Sumpf der Kolonne **RD_{A}** <2> wieder in die Kolonne **RD_{A}** <2> rückgeführt werden. Der Sumpfverdampfer **VS_{A}** <W8> dient dabei insbesondere auch zum Anfahren der Kolonne **RD_{A}** <2>.

Am Kopf der Rektifikationskolonne **RD_{A}** <2> wird Methanolbrüden **S_{DAB}** <24> an einer Entnahmestelle **E_{AK}** <41> abgeführt. Dieser wird dann optional über den Verdichter <12> geleitet und zur Reaktionskolonne **RR_{A}** <1A> rezykliert, wo er als Eduktstrom **S_{AE1}** <1A1> bzw. **S_{BE1}** <1B1> eingesetzt wird. Der Verdichter <12> kann auch anstatt des Verdichters **VD₃₁** <11> eingesetzt werden.

Der Zwischenverdampfer **VZ_{A}** <W9> eröffnet eine Möglichkeit, Energie auf das Gemisch **G_{A}** <21> zu übertragen. Dieses wird der Kolonne **RD_{A}** <2> als Strom **S_{AZ}** <23> entnommen, der Strom **S_{AZ}** <23> im Zwischenverdampfer **VZ_{A}** <W9> erwärmt und dann in die Kolonne **RD_{A}** <2> zurückgeführt.

Ein Strom von Frischmethanol <25> kann dem System über die Rektifikationskolonne **RD_{A}** <2> zugeführt werden. Diesem kann über einen Wärmeübertrager **WT** <W10> Energie zugeführt werden. Das Frischmethanol <25> kann dabei der Rektifikationskolonne **RD_{A}** <2> direkt zugegeben werden.

**S_{DAS}** <22> wird optional über einen Wärmeübertrager **WT** <W15> einer Rektifikationskolonne **RDₓ** <3> zugeleitet. In **RDₓ** <3> ergibt sich dadurch ein Gemisch **Gₓ** <31> umfassend Wasser und Methanol.

In der Rektifikationskolonne **RDₓ** <3> findet die destillative Trennung des Gemisches **Gₓ** <31> in einen Brüdenstrom **S_{XB}** <34> umfassend ROH und einen Sumpfstrom **S_{XS}** <32> umfassend Wasser und ROH statt. Der Sumpfstrom **S_{XS}** <32> wird an der Entnahmestelle **E_{XS}** <52> entnommen und abgeführt. Am Sumpf der Kolonne **RD_{X}** <3> kann der Sumpfstrom **S_{XS}** <32> teilweise wieder über den optionalen Sumpfverdampfer **VS_{X}** <W11> oder den optionalen Sumpfverdampfer **VS_{X}** <W12> in die Kolonne **RD_{X}** <3> rückgeführt werden. Durch den optionalen Sumpfverdampfer **VS_{X}** <WA2> kann gegebenenfalls Energie auf einen Teil von **S_{XS}** <32> übertragen werden, der in **RD_{X}** <3> rezykliert wird. **VZ_{X}** <W12> dient dabei insbesondere auch zum Anfahren der Kolonne **RD_{X}** <3>.

Am Kopf der Rektifikationskolonne **RD_{X}** <3> wird Methanolbrüden **S_{XB}** <34> an einer Entnahmestelle **E_{XK}** <51> entnommen und abgeführt. Dieser wird dann optional über den Verdichter <13> geleitet und gemischt mit **S_{DAB}** <24> zu den Reaktionskolonnen **RR_{A}** <1A> und **RR_{B}** <1B> rezykliert und als Eduktstrom **S_{AE1}** <1A1> bzw. **S_{BE1}** <1B1> eingesetzt.

Der Zwischenverdampfer **VZ_{X}** <W13> eröffnet eine Möglichkeit, Energie auf das Gemisch **G_{X}** <31> zu übertragen. Dieses wird der Kolonne **RD_{X}** <3> als Strom **S_{XZ}** <33> entnommen, letzterer in **VZ_{X}** <W13> erwärmt und dann in die Kolonne **RD_{X}** <3> zurückgeführt.

Ein Strom von Frischmethanol <35> kann dem System über die Rektifikationskolonne **RDₓ** <3> zugeführt werden. Diesem kann über einen Wärmeübertrager **WT** <W14> Energie zugeführt werden. Das Frischmethanol <35> kann dabei der Rektifikationskolonne **RDₓ** <3> direkt zugegeben werden.

Über den Zwischenverdampfer **VZ_{X}** <W13> wird Energie von Heizdampf H₁ <84> auf **S_{XS}** <32> übertragen und somit letztendlich für die destillative Trennung des Gemischs **G_{X}** <31> zur Verfügung gestellt. Dadurch wird ein Heizdampfkondensat **K₁** <81> erhalten, welches in einem Kondensatbehälter <86>, gegebenenfalls mit anderen Kondensaten <88>, gesammelt werden kann. Die Kondensate <88> können sich aus der Beheizung über einen der anderen Wärmeübertrager <WA1> bis <WA6>, <W7>, <W9>, <W10>, wenn die Anlage einen solchen aufweist, ergeben, oder, wenn das erfindungsgemäße Verfahren in der Nähe zu einer entsprechenden Anlage durchgeführt wird, in der Heizdampfkondensat anfällt, von einer solchen Anlagen in den Kondensatbehälter <86> geleitet werden (Strom <88>) In Abbildung 4 wird ein Kondensat <71>, welches sich aus der Beheizung des Sumpfverdampfers **VS_{X}** <W12> mit Heizdampf <74> ergibt, ebenfalls im Kondensatbehälter <86> gesammelt.

Mindestens ein Teil des Kondensats im Sammelbehälter <86> wird zu weiterem Heizdampf **H₂** <82>, dessen Druck **p₂** geringer als der Druck **p₁** von **H₁** <84> ist, entspannt und verdampft. Der dadurch erhaltene Heizdampf **H₂** <82> wird dann mit frischem Heizdampf **H₃** <83>, dessen Druck **p₃** > **p₁** und somit auch **p₃** > **p₂** ist, vermischt. Dadurch wird der Heizdampf/Kondensat/Heizdampf-Kreislauf aus **H₁** <84> / **K₁** <81> / **H₂** <82> vervollständigt, und es wird neuer Heizdampf **H₄** erhalten, der gleich ist wie der Heizdampf **H₁** <84> mit gewünschtem Druck (und somit gewünschter Temperatur) und in der neuen Runde als Heizdampf **H₁** <84> wieder zur Beheizung des Zwischenverdampfers **VZ_{X}** <W13> eingesetzt werden kann.

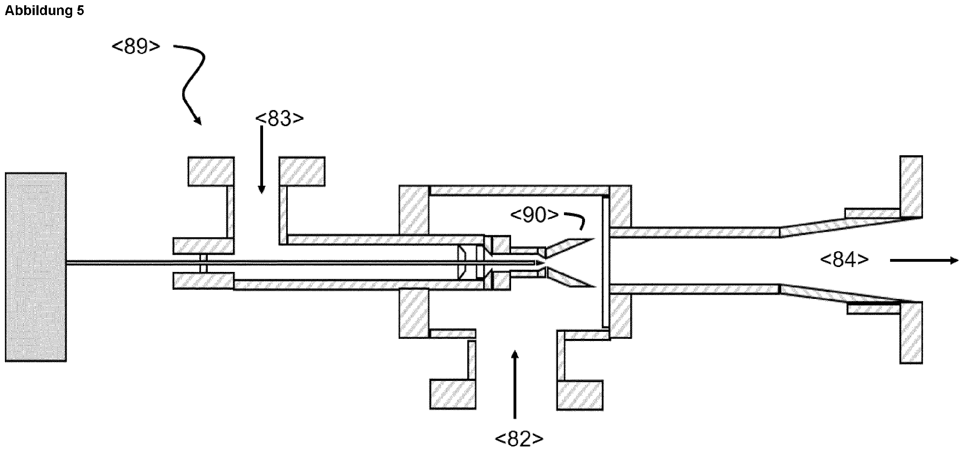

**Abbildung 5** zeigt den schematischen Aufbau eines Dampfstrahlers DS <89>. Der Treibdampf **H₂** <83> ist hier der Heizdampf, insbesondere der Mitteldruckdampf aus dem Dampfnetz. Der Saugdampf **H₂** <82> ist insbesondere der Niederdruckdampf aus dem Kondensatbehälter <86>. Beide werden über die Regeleinheit <90> vermischt und über den Ausgang als Mischdampf **H₄** <84> zum entsprechenden Wärmeübertrager WT, z.B. <W8>, <W9>, <W13> geführt. Über die Regeleinheit <90> können die Mengen von Treibdampf und Saugdampf eingestellt werden, wodurch Druck und damit die Kondensationstemperatur des Mischdampfes **H₄** <84> eingestellt werden können.

### 4. Detaillierte Beschreibung der Erfindung

### 4.1 Schritt (a1) des erfindungsgemäßen Verfahrens

Im Schritt (a1) des erfindungsgemäßen Verfahrens wird ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohproduktgemisch **RP_{A}** umfassend M_{A}OR, Wasser, ROH, M_{A}OH umgesetzt.

Als "Reaktivrektifikationskolonne" wird erfindungsgemäß eine Rektifikationskolonne definiert, in der zumindest in einigen Teilen die Umsetzung nach Schritt (a1) oder Schritt (a2) des erfindungsgemäßen Verfahrens ablaufen. Sie kann auch als "Reaktionskolonne" abgekürzt werden.

In Schritt (a1) des erfindungsgemäßen Verfahrens wird am unteren Ende von **RR_{A}** ein Sumpfproduktstrom **S_{AS}** umfassend ROH und M_{A}OR entnommen. Am oberen Ende von **RR_{A}** wird ein Brüdenstrom **S_{AB}** umfassend Wasser und ROH entnommen.

"Brüdenstrom" bedeutet, dass es sich bei dem jeweiligen Strom um einen gasförmigen Strom handelt.

R ist im erfindungsgemäßen Verfahren ein C₁ bis C₆-Kohlenwasserstoffrest, bevorzugt ausgewählt aus der Gruppe bestehend aus Methyl, Ethyl, *n*-Propyl, iso-Propyl, *n*-Butyl, *sec*-Butyl, *iso*-Butyl, *tert*-Butyl, Isomeren des Pentyls, wie beispielsweise *n*-Pentyl, bevorzugter ausgewählt aus der Gruppe bestehend aus Methyl, Ethyl, *n*-Propyl, *iso*-Propyl, noch bevorzugter ausgewählt aus der Gruppe bestehend aus Methyl, Ethyl. Besonders bevorzugt ist R Methyl und ROH demnach Methanol.

M_{A} ist ausgewählt aus Natrium, Kalium, und bevorzugt Natrium.

Der Eduktstrom **S_{AE1}** umfasst ROH. In einer bevorzugten Ausführungsform liegt der Massenanteil von ROH in **S_{AE1},** bezogen auf die gesamte Masse des Eduktstroms **S_{AE1},** bei ≥ 95 Gew.-%, noch bevorzugter bei ≥ 99 Gew.-%, wobei **S_{AE1}** ansonsten insbesondere Wasser aufweist.

Der in Schritt (a1) des erfindungsgemäßen Verfahrens als Eduktstrom **S_{AE1}** eingesetzte Alkohol ROH kann auch handelsüblicher Alkohol mit einem Alkoholmassenanteil, bezogen auf die gesamte Masse des Eduktstroms **S_{AE1},** von mehr als 99.8 Gew.-% und einem Massenanteil an Wasser, bezogen auf die gesamte Masse des Eduktstroms **S_{AE1},** von bis zu 0.2 Gew.-% sein.

Der Eduktstrom **S_{AE1}** wird bevorzugt dampfförmig zugegeben.

Der Eduktstrom **S_{AE2}** umfasst M_{A}OH, bevorzugt in einem Massenanteil von 10 bis 75 Gew.-%, bevorzugter 20 bis 55 Gew.-%, bevorzugter 48.5 Gew.-%, jeweils bezogen auf die Gesamtmasse des Stromes **S_{AE2}**. In einer bevorzugten Ausführungsform umfasst **S_{AE2}** neben M_{A}OH mindestens eine weitere Verbindung ausgewählt aus Wasser, ROH. Noch bevorzugter umfasst **S_{AE2}** neben M_{A}OH auch Wasser, dann handelt es sich bei **S_{AE2}** um eine wässrige Lösung von M_{A}OH.

Schritt (a1) des erfindungsgemäßen Verfahrens wird in einer Reaktivrektifikationskolonne **RR_{A}** durchgeführt. Schritt (a2) des erfindungsgemäßen Verfahrens wird in einer Reaktivrektifikationskolonne **RR_{B}** durchgeführt.

Bevorzugt enthält die Reaktionskolonne **RR_{A}** bzw. **RR_{B}** Einbauten. Geeignete Einbauten sind zum Beispiel Böden, strukturierte Packungen oder unstrukturierte Packungen (Füllkörper). Wenn die Reaktionskolonne **RR_{A}** bzw. **RR_{B}** Böden enthält, so sind Glockenböden, Ventilböden, Tunnelböden, Thormann-Böden, Kreuzschlitzglockenböden oder Siebböden geeignet. Wenn die Reaktionskolonne **RR_{A}** bzw. **RR_{B}** Böden enthält, so werden vorzugsweise solche Böden gewählt, bei denen maximal 5 Gew.-%, bevorzugt weniger als 1 Gew.-% der Flüssigkeit durch die jeweiligen Böden durchregnen. Die zur Minimierung des Durchregnens der Flüssigkeit erforderlichen konstruktiven Maßnahmen sind dem Fachmann geläufig. Bei Ventilböden werden zum Beispiel besonders dicht schließende Ventilbauarten gewählt. Durch Verringerung der Zahl der Ventile lässt sich zudem die Dampfgeschwindigkeit in den Bodenöffnungen auf das Doppelte des Wertes, der üblicherweise eingestellt wird, erhöhen. Bei Einsatz von Siebböden ist es besonders günstig, die Durchmesser der Bodenöffnungen zu verringern und die Zahl der Öffnungen beizubehalten.

Bei Einsatz von strukturierten oder unstrukturierten Packungen sind strukturierte Packungen im Hinblick auf die gleichmäßige Verteilung der Flüssigkeit bevorzugt.

Bei Kolonnen mit unstrukturierten Packungen, insbesondere mit Füllkörpern, und bei Kolonnen mit strukturierten Packungen kann die gewünschte Charakteristik der Flüssigkeitsverteilung dadurch erzielt werden, dass in dem dem Kolonnenmantel benachbarten Randbereich des Kolonnenquerschnitts, der etwa 2 bis 5 % des gesamten Kolonnenquerschnitts entspricht, die Flüssigkeitsberieselungsdichte gegenüber den übrigen Querschnittsbereichen um bis zu 100 %, bevorzugt um 5 bis 15 %, verringert wird. Dies lässt sich zum Beispiel durch die gezielte Verteilung der Abtropfstellen der Flüssigkeitsverteiler oder deren Bohrungen mit einfachen Mitteln erreichen.

Um Tropfenmitriss zu vermeiden, können die Kolonnen außerdem entsprechende dem Fachmann bekannte Einbauten umfassen, zum Beispiel Drahtgestrick-Tropfenabscheider. Beispiele für solche Tropfenabscheider, wie jene unter dem Namen "KnitMesh V-MISTER^{™}- Technologie" von der Firma Sulzer vertriebene, sind in der Broschüre "Gas/Liquid Separation Technology" E10508 en 4.2018, Copyright ^{©} Sulzer Ltd 2018 beschrieben, abrufbar online unter: https://www.sulzer.com/-/media/files/products/separation-technology/feed-inletdevices/gas_liquid separation technology.ashx?la=en.

Das erfindungsgemäße Verfahren kann sowohl kontinuierlich als auch diskontinuierlich erfolgen. Bevorzugt erfolgt es kontinuierlich.

"Umsetzung eines Eduktstroms **S_{AE1}** umfassend ROH mit einem Eduktstrom **S_{AE2}** umfassend M_{A}OH im Gegenstrom" wird erfindungsgemäß insbesondere dadurch gewährleistet, dass der Zugabepunkt mindestens eines Teils des Eduktstroms **S_{AE1}** umfassend ROH in Schritt (a1) an der Reaktionskolonne **RR_{A}** unterhalb des Zugabepunktes des Eduktstroms **S_{AE2}** umfassend M_{A}OH liegt.

Die Reaktionskolonne **RR_{A}** umfasst vorzugsweise mindestens 2, insbesondere 15 bis 40 theoretische Böden zwischen dem Zugabepunkt des Eduktstroms **S_{AE1}** und dem Zugabepunkt des Eduktstroms **S_{AE2}**.

Insbesondere wird im unteren Bereich der Reaktionskolonne **RR_{A}** dampfförmig der Eduktstrom **S_{AE1}** umfassend ROH zugeführt. Schritt (a1) des erfindungsgemäßen Verfahrens umfasst auch den Fall, dass ein Teil des Eduktstroms **S_{AE1}** umfassend ROH zwar unterhalb des Zugabepunktes des Eduktstroms **S_{AE2}** umfassend Alkalilauge M_{A}OH, aber dennoch am oberen Ende oder im Bereich des oberen Endes der Reaktionskolonne **RR_{A}** dampfförmig zugegeben wird. Dadurch können die Abmessungen im unteren Bereich der Reaktionskolonne **RR_{A}** verringert werden. Wenn ein Teil des Eduktstroms **S_{AE1}** umfassend ROH, insbesondere Methanol, am oberen Ende oder im Bereich des oberen Endes der Reaktionskolonne **RR_{A}** insbesondere dampfförmig zugegeben wird, so wird nur eine Teilmenge von 10 bis 70 Gew.-%, bevorzugt von 30 bis 50 Gew.-% [jeweils bezogen auf die Gesamtmenge des in Schritt (a1) eingesetzten Alkohols ROH] am unteren Ende der Reaktionskolonne **RR_{A}** eingespeist und die restliche Teilmenge in einem Einzelstrom oder auf mehrere Teilströme verteilt, bevorzugt 1 bis 10 theoretische Böden, besonders bevorzugt 1 bis 3 theoretische Böden unterhalb des Zugabepunktes des Eduktstroms **S_{AE2}** umfassend M_{A}OH dampfförmig zugegeben.

In der Reaktionskolonne **RR_{A}** setzt sich dann der Eduktstrom **S_{AE1}** umfassend ROH mit dem Eduktstrom **S_{AE2}** umfassend M_{A}OH gemäß der vorstehend beschriebenen Reaktion <1> zu M_{A}OR und H₂O um, wobei, da es sich um eine Gleichgewichtsreaktion handelt, diese Produkte in Mischung mit den Edukten ROH und M_{A}OH vorliegen. Demnach wird in Schritt (a1) des erfindungsgemäßen Verfahrens in der Reaktionskolonne **RR_{A}** ein Rohproduktgemisch **RP_{A}** erhalten, welches neben den Produkten M_{A}OR und Wasser auch noch ROH und M_{A}OH umfasst.

Am unteren Ende von **RR_{A}** wird dann der Sumpfproduktstrom **S_{AS}** ("**S_{AS}**" kann auch als "**S_{AP}**" bezeichnet werden) umfassend ROH und M_{A}OR entnommen.

Am oberen Ende von **RR_{A}** wird dann der noch Wasser enthaltenden Alkoholstrom, vorstehend als "Brüdenstrom **S_{AB}** umfassend Wasser und ROH" bezeichnet, entnommen.

Die Menge des vom Eduktstrom **S_{AE1}** umfassten Alkohols ROH wird vorzugsweise so gewählt, dass dieser gleichzeitig als Lösungsmittel für das im Sumpfproduktstrom **S_{AS}** erhaltene Alkalialkoholat M_{A}OR dient.

In einer bevorzugten Ausführungsform des erfindungsgemäßen Verfahrens, und insbesondere in den Fällen, in denen **S_{AE2}** neben M_{A}OH auch Wasser umfasst, beträgt das Verhältnis aus dem Gesamtgewicht (Massen; Einheit: kg) an in Schritt (a1) als Eduktstrom **S_{AE1}** eingesetztem Alkohol ROH zum Gesamtgewicht (Massen; Einheit: kg) an in Schritt (a1) als Eduktstrom **S_{AE2}** eingesetzten M_{A}OH 2 : 1 bis 50 : 1, bevorzugter 5 : 1 bis 48 : 1, noch bevorzugter 10 : 1 bis 35 : 1, noch mehr bevorzugter 13 : 1 bis 30 : 1.

Die Reaktionskolonne **RR_{A}** wird mit oder ohne, vorzugsweise ohne, Rücklauf betrieben.

"Ohne Rücklauf" bedeutet, dass der am oberen Ende von **RR_{A}** entnommene Brüdenstrom **S_{AB}** umfassend Wasser und ROH bzw. der der im optionalen Schritt (a2) am oberen Ende von **RR_{B}** entnommene Brüdenstrom **S_{BB}** umfassend Wasser und ROH der Rektifikationskolonne **RD_{A}** gemäß Schritt (b) vollständig zugeführt wird. Der Brüdenstrom **S_{AB}** bzw. der im optionalen Schritt (a2) entnommene Brüdenstrom **S_{AB}** wird der ersten Rektifikationskolonne **RD_{A}** dabei vorzugsweise dampfförmig zugeführt.

"Mit Rücklauf" bedeutet, dass der am oberen Ende der jeweiligen Kolonne, in Schritt (a1) der Reaktionskolonne **RR_{A},** entnommene Brüdenstrom **S_{AB}** umfassend Wasser und ROH und im optionalen Schritt (a2) der Reaktionskolonne **RR_{B},** entnommene Brüdenstrom **S_{BB}** umfassend Wasser und ROH nicht vollständig von **RR_{A}** bzw. **RR_{B}** abgeführt wird, also nicht vollständig in die Rektifikationskolonne **RD_{A}** geleitet wird, sondern teilweise wieder als Rücklauf der jeweiligen Kolonne, in Schritt (a1) **S_{AB}** also der Reaktionskolonne **RR_{A},** im optionalen Schritt (a2) **S_{BB}** also der Reaktionskolonne **RR_{B},** zugeführt wird. In den Fällen, in denen ein solcher Rücklauf eingestellt wird, beträgt das Rücklaufverhältnis dabei bevorzugt 0.005 bis 0.99, bevorzugter 0.01 bis 0.9, noch bevorzugter 0.02 bis 0.34, besonders bevorzugt 0.025 bis 0.27 und ganz besonders bevorzugt 0.03 bis 0.24. Ein Rücklauf kann dadurch eingestellt werden, dass am Kopf der jeweiligen Kolonne, in Schritt (a1) der Reaktionskolonne **RR_{A}** ein Kondensator **b** und im optionalen Schritt (a2) der Reaktionskolonne **RR_{B}** ein Kondensator **K_{RRB}**, angebracht wird, in welchem der Brüdenstrom **S_{AB}** bzw. **S_{BB}** mindestens teilweise kondensiert wird und der jeweiligen Kolonne **RR_{A}** bzw. **RR_{B}** wieder zugeführt wird. Unter einem Rücklaufverhältnis wird allgemein und im Sinne dieser Erfindung das Verhältnis des Massenstroms (kg/h) verstanden, der der jeweiligen Kolonne in flüssiger Form zurückgeführt wird (Rücklauf) zu dem Massenstrom (kg/h), der in flüssiger Form (Destillat) oder gasförmiger Form (Brüden) von der jeweiligen Kolonne abgeführt wird.

In der Ausführungsform, in der an der Reaktionskolonne **RR_{A}** ein Rücklauf eingestellt wird, kann das in Schritt (a1) als Eduktstrom **S_{AE2}** eingesetzte Alkalihydroxid M_{A}OH auch mindestens teilweise mit dem Rücklaufstrom vermischt werden und die resultierende Mischung so dem Schritt (a1) zugeführt werden.

Der Schritt (a1) des erfindungsgemäßen Verfahrens wird insbesondere bei einer Temperatur **T_{RRA}** im Bereich von 45 °C bis 190 °C, bevorzugt 47 °C bis 170 °C, bevorzugter 60 °C bis 150 °C durchgeführt und insbesondere bei einem Druck **p_{RRA}** von 0.5 bar bis 40 bar, bevorzugt im Bereich von 0.75 bar bis 20 bar, bevorzugter im Bereich von 0.9 bar bis 10 bar, bevorzugter im Bereich von 1 bar bis 7 bar, noch bevorzugter bei 1 bar bis 5 bar, durchgeführt.

Die Reaktionskolonne **RR_{A}** umfasst in einer bevorzugten Ausführungsform mindestens einen Verdampfer, der insbesondere aus Zwischenverdampfern **VZ_{RRA}** und Sumpfverdampfern **VS_{RRA}** ausgewählt ist, noch bevorzugter mindestens einen Sumpfverdampfer **VS_{RRA}**.

Als "Zwischenverdampfer" **VZ_{RRA}** bzw. **VZ_{RRB}** werden erfindungsgemäß Verdampfer bezeichnet, die sich oberhalb des Sumpfs der jeweiligen Kolonne, insbesondere oberhalb des Sumpfs der Reaktionskolonne **RR_{A}** bzw. **RR_{B},** befinden. In ihnen wird insbesondere Rohproduktgemisch **RP_{A}** bzw. **RP_{B}** verdampft.

Als "Sumpfverdampfer" **VS_{RRA}** und **VS_{RRB}** werden erfindungsgemäß Verdampfer bezeichnet, die den Sumpf der jeweiligen Kolonne, insbesondere den Sumpf der Reaktionskolonne **RR_{A}** bzw. **RR_{B},** beheizen. In ihnen wird Sumpfproduktstrom **S_{AS}** bzw. **S_{BS}** ("**S_{BS}**" kann auch als "**S_{BP}**" bezeichnet werden) mindestens teilweise verdampft.

Ein Verdampfer ist üblicherweise außerhalb der jeweiligen Reaktionskolonne oder Rektifikationskolonne angeordnet. Über einen Abzug oder "Entnahmestelle" aus der Kolonne wird das im Verdampfer zu verdampfende Gemisch abgezogen und dem mindestens einen Verdampfer zugeführt.

Im Falle der Reaktionskolonne **RR_{A}** bzw. **RR_{B}** wird, bei der Zwischenverdampfung des Rohproduktgemischs **RP_{A}** bzw. **RP_{B}**, dieses als Strom **S_{RRAZ}** bzw. **S_{RRBZ}** abgezogen und dem mindestens einen Zwischenverdampfer **VZ_{RRA}** bzw. **VZ_{RRB}** zugeführt. Über einen Zulauf oder "Zugabestelle" wird das verdampfte Gemisch, gegebenenfalls mit einem Restanteil Flüssigkeit, wieder in die jeweilige Kolonne **RR_{A}** bzw. **RR_{B}** zurückgeführt. Wenn der Verdampfer ein Zwischenverdampfer ist, es sich also insbesondere um einen Zwischenverdampfer **VZ_{RRA}** bzw. **VZ_{RRB}** handelt, ist der Abzug, über den das jeweilige Gemisch abgezogen und dem Verdampfer zugeführt wird, ein Seitenabzug und der Zulauf, über den das verdampfte jeweilige Gemisch der Kolonne wieder zugeführt wird, ein Seitenzulauf. Wenn der Verdampfer ein Sumpfverdampfer ist, also den Kolonnensumpf beheizt, es sich also insbesondere um einen Sumpfverdampfer **VS_{RRA}** bzw. **VS_{RRB}** handelt, so wird zumindest ein Teil des Sumpfabzugsstroms, insbesondere **S_{AS}** bzw. **S_{BS}**, dem entsprechenden Sumpfverdampfer zugeführt, verdampft und im Bereich des Sumpfs wieder in die jeweilige Kolonne zurückgeführt.

Alternativ ist es jedoch auch möglich, zum Beispiel auf einem geeigneten Boden bei Einsatz eines Zwischenverdampfers oder im Sumpf der jeweiligen Kolonne Rohre auszubilden, die von dem entsprechenden Wärmemedium, wie zum Beispiel **H₁** oder **H₄**, durchströmt werden. In diesem Fall erfolgt die Verdampfung auf dem Boden bzw. im Sumpf der Kolonne. Bevorzugt ist es jedoch, den Verdampfer außerhalb der jeweiligen Kolonne anzuordnen.

Geeignete Verdampfer VD, die als Zwischenverdampfer VZ und Sumpfverdampfer VS eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als wärmeübertragendes Bauteil für den Verdampfer wird bei Naturumlaufverdampfern und Zwangsumlaufverdampfern üblicherweise ein Rohrbündel oder Plattenapparat eingesetzt. Bei Einsatz eines Rohrbündelüberträgers kann das Wärmemedium, wie zum Beispiel **H₁** oder **H₄**, entweder durch die Rohre strömen und das zu verdampfende Gemisch die Rohre umströmen oder aber das Wärmemedium umströmt die Rohre und das zu verdampfende Gemisch durchströmt die Rohre. Bei einem Fallfilmverdampfer wird das zu verdampfende Gemisch üblicherweise als dünner Film auf der Innenseite eines Rohres zugegeben und das Rohr wird von außen beheizt. Im Unterschied zu einem Fallfilmverdampfer ist in einem Dünnschichtverdampfer zusätzlich ein Rotor mit Wischern vorgesehen, der die zu verdampfende Flüssigkeit auf der Innenwand des Rohres zu einem dünnen Film verteilt.

Neben den genannten kann aber auch jede beliebige andere, dem Fachmann bekannte Verdampferbauart, die sich zum Einsatz an einer Rektifikationskolonne bzw. Reaktionskolonne eignet, eingesetzt werden.

Wenn die Reaktionskolonne **RR_{A}** bzw. Reaktionskolonne **RR_{B}** einen Zwischenverdampfer **VZ_{RRA}** bzw. **VZ_{RRB}** aufweist, ist es bevorzugt, wenn der jeweilige Zwischenverdampfer im Falle der Reaktionskolonne **RR_{A}** im Bereich der Zulaufstelle des Eduktstroms **S_{AE1}** bzw. im Falle der Reaktionskolonne **RR_{B}** im Bereich der Zulaufstelle des Eduktstroms **S_{BE1}** angeordnet ist. Dadurch kann ein überwiegender Teil der Heizenergie durch den Zwischenverdampfer **VZ_{RRA}** bzw. **VZ_{RRB}** eingebracht werden. So ist es zum Beispiel möglich, über 80% der Energie über den Zwischenverdampfer einzubringen. Erfindungsgemäß wird der Zwischenverdampfer **VZ_{RRA}** bzw. **VZ_{RRB}** vorzugsweise so angeordnet und/oder gestaltet, dass mit diesem mehr als 50 %, insbesondere mehr als 75 % der für die Reaktivrektifikation benötigten Gesamtenergie eingebracht werden.

Wenn die Reaktionskolonne **RR_{A}** bzw. Reaktionskolonne **RR_{B}** einen Zwischenverdampfer **VZ_{RRA}** bzw. **VZ_{RRB}** aufweist, ist es außerdem vorteilhaft, wenn der Zwischenverdampfer so angeordnet ist, dass die Reaktionskolonne **RR_{A}** bzw. **RR_{B}** unterhalb des Zwischenverdampfers 1 bis 50, bevorzugt 1 bis 40, theoretische Böden aufweist und oberhalb des Zwischenverdampfers 1 bis 200, bevorzugt 1 bis 40, theoretische Böden aufweist. Insbesondere ist es bevorzugt, wenn die Reaktionskolonne **RR_{A}** bzw. **RR_{B}** dann unterhalb des Zwischenverdampfers 2 bis 10 theoretische Böden aufweist und oberhalb des Zwischenverdampfers 20 bis 50 theoretische Böden aufweist.

Wenn die Reaktionskolonne **RR_{A}** bzw. Reaktionskolonne **RR_{B}** einen Zwischenverdampfer **VZ_{RRA}** bzw. **VZ_{RRB}** aufweist, ist es außerdem vorteilhaft, wenn der Seitenabzug (also die "Entnahmestelle **E_{RRA}"** an der Reaktionskolonne **RR_{A}** bzw. die "Entnahmestelle **E_{RRB}"** an der Reaktionskolonne **RR_{B}**), über den das Rohproduktgemisch **RP_{A}** bzw. **RP_{B}** dem Zwischenverdampfer **VZ_{RRA}** bzw. **VZ_{RRB}** zugeführt wird, und der Seitenzulauf (also die "Zugabestelle **Z_{RRA}"** an der Reaktionskolonne **RR_{A}** bzw. die "Zugabestelle **Z_{RRB}"** an der Reaktionskolonne **RR_{B}**), über den das verdampfte Rohproduktgemisch **RP_{A}** bzw. **RP_{B}** aus dem Zwischenverdampfer **VZ_{RRA}** bzw. **VZ_{RRB}** wieder der jeweiligen Reaktionskolonne **RR_{A}** bzw. **RR_{B}** zugeführt wird, höchstens 2 Böden voneinander entfernt sind, bevorzugt zwischen den gleichen Böden der Reaktionskolonne **RR_{A}** bzw. Reaktionskolonne **RR_{B}** positioniert ist. Es ist jedoch auch möglich, dass Seitenabzug und Seitenzulauf auf unterschiedlicher Höhe liegen.

Es ist weiterhin vorteilhaft, dass, wenn die Reaktionskolonne **RR_{A}** bzw. die Reaktionskolonne **RR_{B}** einen Zwischenverdampfer **VZ_{RRA}** bzw. **VZ_{RRB}** aufweist, dass die "Entnahmestelle **E_{RRA}"** an der Reaktionskolonne **RR_{A}** bzw. die "Entnahmestelle **E_{RRB}**" an der Reaktionskolonne **RR_{B},** und die "Zugabestelle **Z_{RRA}**" an der Reaktionskolonne **RR_{A}** bzw. die "Zugabestelle **Z_{RRB}**" an der Reaktionskolonne **RR_{B},** unterhalb der Zugabestellen von **S_{AE2}** bzw. **S_{BE2}** liegen.

In einer bevorzugten Ausführungsform ist bei Einsatz eines Zwischenverdampfers **VZ_{RRA}** bzw. **VZ_{RRB}** in **RR_{A}** bzw. **RR_{B}** der Durchmesser der Reaktionskolonne **RR_{A}** bzw. **RR_{B}** oberhalb des Zwischenverdampfers **RR_{A}** bzw. **RR_{B}** größer als der Durchmesser der Reaktionskolonne **RR_{A}** bzw. **RR_{B}** unterhalb des Zwischenverdampfers **VZ_{RRA}** bzw. **VZ_{RRB}**. Dies hat den Vorteil, dass Investitionskosten gespart werden können.

In einem solchen Zwischenverdampfer **VZ_{RRA}** bzw. **VZ_{RRB}** kann in der Reaktionskolonne **RR_{A}** befindliches, flüssiges Rohproduktgemisch **RP_{A}** umfassend M_{A}OR, Wasser, ROH, M_{A}OH bzw. in der Reaktionskolonne **RR_{B}** befindliches, flüssiges Rohproduktgemisch **RP_{B}** umfassend M_{B}OR, Wasser, ROH, M_{B}OH in den gasförmigen Zustand überführt werden.

Durch die Anordnung von einem oder mehreren Zwischenverdampfern **VZ_{RRA}** im oberen Bereich der Reaktionskolonne **RR_{A}** bzw. von einem oder mehreren Zwischenverdampfern **VZ_{RRB}** im oberen Bereich der Reaktionskolonne **RR_{B}** können die Abmessungen im unteren Bereich der Reaktionskolonne **RR_{A}** bzw. **RR_{B}** verringert werden. Bei der Ausführungsform mit mindestens einem, bevorzugt mehreren Zwischenverdampfern **VZ_{RRA}** bzw. **VZ_{RRB}** ist es auch möglich, Teilströme des ROH in flüssiger Form im oberen Bereich der Reaktionskolonne **RR_{A}** bzw. **RR_{B}** zuzuführen.

In Schritt (a1) des erfindungsgemäßen Verfahrens wird am unteren Ende der Reaktionskolonne **RR_{A}** ein Sumpfproduktstrom **S_{AS}** umfassend ROH und M_{A}OR entnommen.

Es ist bevorzugt, dass die Reaktionskolonne **RR_{A}** mindestens einen Sumpfverdampfer **VS_{RRA}** aufweist, über den Sumpfproduktstrom **S_{AS}** dann mindestens teilweise geleitet wird

Sumpfverdampfer sind erfindungsgemäß am Sumpf der Reaktionskolonne **RR_{A}** bzw. **RR_{B}** angeordnet und werden dann als "**VS_{RRA}**" bzw. "**VS_{RRB}"** bezeichnet. In einem solchen Sumpfverdampfer kann in der Reaktionskolonne **RR_{A}** bzw. **RR_{B}** befindlicher Sumpfproduktstrom **S_{AS}** bzw. **S_{BS}** geleitet und ROH mindestens teilweise daraus entfernt werden, wodurch der Massenanteil an M_{A}OR bzw. M_{B}OR im jeweiligen Sumpfstrom gezielt eingestellt werden kann.

Bevorzugt weist der in Schritt (a1) entnommene Sumpfproduktstrom **S_{AS}** einen Massenanteil von M_{A}OR in ROH im Bereich 1 bis 50 Gew.-%, bevorzugt 5 bis 32 Gew.-%, bevorzugter 10 bis 32 Gew.-%, noch bevorzugter 15 bis 32 Gew.-% auf, jeweils bezogen auf die Gesamtmasse von **S_{AS}**.

Der Massenanteil an Restwasser in **S_{AS}** liegt dabei bevorzugt bei < 1 Gew.-%, bevorzugt < 0.5 Gew.-%, bevorzugter < 0.2 Gew.-%, bezogen auf die Gesamtmasse von **S_{AS}**.

Der Massenanteil an Edukt M_{A}OH in **S_{AS}** liegt dabei bevorzugt bei < 1 Gew.-%, bevorzugt < 0.5 Gew.-%, bevorzugter < 0.2 Gew.-%, bezogen auf die Gesamtmasse von **S_{AS}**.

### 4.2 Schritt (a2) des erfindungsgemäßen Verfahrens (optional)

Schritt (a2) wird erfindungsgemäß optional durchgeführt. Im optionalen Schritt (a2), der gleichzeitig mit und räumlich getrennt von Schritt (a1) des erfindungsgemäßen Verfahrens abläuft, wird ein Eduktstrom **S_{BE1}** umfassend ROH mit einem Eduktstrom **S_{BE2}** umfassend M_{B}OH im Gegenstrom in einer Reaktivrektifikationskolonne **RR_{B}** zu einem Rohproduktgemisch **RP_{B}** umfassend M_{B}OR, Wasser, ROH, M_{B}OH umgesetzt.

Im optionalen Schritt (a2) des erfindungsgemäßen Verfahrens wird am unteren Ende von **RR_{B}** ein Sumpfproduktstrom **S_{BS}** umfassend ROH und M_{B}OR entnommen. Am oberen Ende von **RR_{B}** wird ein Brüdenstrom **S_{BB}** umfassend Wasser und ROH entnommen.

M_{B} ist ausgewählt aus Natrium, Kalium, und bevorzugt Kalium.

Der Eduktstrom **S_{BE1}** umfasst ROH. In einer bevorzugten Ausführungsform liegt der Massenanteil von ROH in **S_{BE1}**, bezogen auf die gesamte Masse des Eduktstroms **S_{BE1}**, bei ≥ 95 Gew.-%, noch bevorzugter bei ≥ 99 Gew.-%, wobei **S_{BE1}** ansonsten insbesondere Wasser aufweist.

Der im optionalen Schritt (a2) des erfindungsgemäßen Verfahrens als Eduktstrom **S_{BE1}** eingesetzte Alkohol ROH kann auch handelsüblicher Alkohol mit einem Alkoholmassenanteil, bezogen auf die gesamte Masse des Eduktstroms **S_{BE1}**, von mehr als 99.8 Gew.-% und einem Massenanteil an Wasser, bezogen auf die gesamte Masse des Eduktstroms **S_{BE1}**, von bis zu 0.2 Gew.-% sein.

Der Eduktstrom **S_{BE1}** wird bevorzugt dampfförmig zugegeben.

Der Eduktstrom **S_{BE2}** umfasst M_{B}OH, bevorzugt in einem Massenanteil von 10 bis 75 Gew.-%, bevorzugter 20 bis 55 Gew.-%, bevorzugter 48 bis 52 Gew.-%, jeweils bezogen auf die Gesamtmasse des Stromes **S_{BE2}**. In einer bevorzugten Ausführungsform umfasst **S_{BE2}** neben M_{B}OH mindestens eine weitere Verbindung ausgewählt aus Wasser, ROH. Noch bevorzugter umfasst **S_{BE2}** neben M_{B}OH Wasser, dann handelt es sich bei **S_{BE2}** um eine wässrige Lösung von M_{B}OH.

Schritt (a2) des erfindungsgemäßen Verfahrens wird in einer Reaktivrektifikationskolonne (oder "Reaktionskolonne") **RR_{B}** durchgeführt. Bevorzugte Ausführungsformen der Reaktionskolonne **RR_{B}** sind im Abschnitt 4.1 beschrieben.

"Umsetzung eines Eduktstroms **S_{BE1}** umfassend ROH mit einem Eduktstrom **S_{BE2}** umfassend M_{B}OH im Gegenstrom" wird erfindungsgemäß insbesondere dadurch gewährleistet, dass der Zugabepunkt mindestens eines Teils des Eduktstroms **S_{BE1}** umfassend ROH im optionalen Schritt (a2) an der Reaktionskolonne **RR_{B}** unterhalb des Zugabepunktes des Eduktstroms **S_{BE2}** umfassend M_{B}OH liegt.

Die Reaktionskolonne **RR_{B}** umfasst vorzugsweise mindestens 2, insbesondere 15 bis 40 theoretische Böden zwischen dem Zugabepunkt des Eduktstroms **S_{BE1}** und dem Zugabepunkt des Eduktstroms **S_{BE2}**.

Insbesondere wird im unteren Bereich der Reaktionskolonne **RR_{B}** dampfförmig der Eduktstrom **S_{BE1}** umfassend ROH zugeführt. Der optionale Schritt (a2) des erfindungsgemäßen Verfahrens umfasst auch den Fall, dass ein Teil des Eduktstroms **S_{BE1}** umfassend ROH zwar unterhalb des Zugabepunktes des Eduktstroms **S_{BE2}** umfassend Alkalilauge M_{B}OH, aber dennoch am oberen Ende oder im Bereich des oberen Endes der Reaktionskolonne **RR_{B}** dampfförmig zugegeben wird. Dadurch können die Abmessungen im unteren Bereich der Reaktionskolonne **RR_{B}** verringert werden. Wenn ein Teil des Eduktstroms **S_{BE1}** umfassend ROH, insbesondere Methanol, am oberen Ende oder im Bereich des oberen Endes der Reaktionskolonne **RR_{B}** insbesondere dampfförmig zugegeben wird, so wird nur eine Teilmenge von 10 bis 70 Gew.-%, bevorzugt von 30 bis 50 Gew.-% [jeweils bezogen auf die Gesamtmenge des im optionalen Schritt (a2) eingesetzten Alkohols ROH] am unteren Ende der Reaktionskolonne **RR_{B}** eingespeist und die restliche Teilmenge in einem Einzelstrom oder auf mehrere Teilströme verteilt, bevorzugt 1 bis 10 theoretische Böden, besonders bevorzugt 1 bis 3 theoretische Böden unterhalb des Zugabepunktes des Eduktstroms **S_{BE2}** umfassend M_{B}OH dampfförmig zugegeben.

In der Reaktionskolonne **RR_{B}** setzt sich dann der Eduktstrom **S_{BE1}** umfassend ROH mit dem Eduktstrom **S_{BE2}** umfassend M_{B}OH gemäß der vorstehend beschriebenen Reaktion <1> zu M_{B}OR und H₂O um, wobei, da es sich um eine Gleichgewichtsreaktion handelt, diese Produkte in Mischung mit den Edukten ROH und M_{B}OH vorliegen. Demnach wird im optionalen Schritt (a2) des erfindungsgemäßen Verfahrens in der Reaktionskolonne **RR_{B}** ein Rohproduktgemisch **RP_{B}** erhalten, welches neben den Produkten M_{B}OR und Wasser auch noch ROH und M_{B}OH umfasst.

Am unteren Ende von **RR_{B}** erhält und entnimmt man dann den Sumpfproduktstrom **S_{BS}** ("**S_{BS}**" kann auch als "**S_{BP}**" bezeichnet werden) umfassend ROH und M_{B}OR.

Am oberen Ende von **RR_{B}** wird dann der noch Wasser enthaltende Alkoholstrom, vorstehend als "Brüdenstrom **S_{BB}** umfassend Wasser und ROH" bezeichnet, entnommen.

Die Menge des vom Eduktstrom **S_{BE1}** umfassten Alkohols ROH wird vorzugsweise so gewählt, dass dieser gleichzeitig als Lösungsmittel für das im Sumpfproduktstrom **S_{BP}** erhaltene Alkalialkoholat M_{B}OR dient.

In einer bevorzugten Ausführungsform des optionalen Schritts (a2) des erfindungsgemäßen Verfahrens, und insbesondere in den Fällen, in denen **S_{BE2}** neben M_{B}OH auch Wasser umfasst, beträgt das Verhältnis aus dem Gesamtgewicht (Massen; Einheit: kg) an in Schritt (a2) als Eduktstrom **S_{BE1}** eingesetztem Alkohol ROH zum Gesamtgewicht (Massen; Einheit: kg) an in Schritt (a2) als Eduktstrom **S_{BE2}** eingesetzten M_{B}OH 2 : 1 bis 50 : 1, bevorzugter 5 : 1 bis 48 : 1, noch bevorzugter 10 : 1 bis 35 : 1, noch mehr bevorzugter 13 : 1 bis 30 : 1.

Dieser Brüdenstrom **S_{BB}** umfassend Wasser und ROH wird dem Schritt (b) des erfindungsgemäßen Verfahrens zugeführt. Dabei wird er, bevor er dem Schritt (b) des erfindungsgemäßen Verfahrens zugeführt wird, mit **S_{AB}** vermischt oder getrennt von **S_{AB}** dem Schritt (b) des erfindungsgemäßen Verfahrens zugeführt. Bevorzugt wird Brüdenstrom **S_{BB}** mit **S_{AB}** vermischt und dann der erhaltene Mischbrüdenstrom dem Schritt (b) des erfindungsgemäßen Verfahrens zugeführt.

Die Reaktionskolonne **RR_{B}** wird mit oder ohne, vorzugsweise ohne Rücklauf betrieben.

In der Ausführungsform, in der an der Reaktionskolonne **RR_{B}** ein Rücklauf eingestellt wird, kann das im optionalen Schritt (a2) als Eduktstrom **S_{BE2}** eingesetzte Alkalihydroxid M_{B}OH auch mindestens teilweise mit dem Rücklaufstrom vermischt werden und die resultierende Mischung so dem optionalen Schritt (a2) zugeführt werden.

Der optionale Schritt (a2) des erfindungsgemäßen Verfahrens wird insbesondere bei einer Temperatur **T_{RRB}** im Bereich von 45 °C bis 190 °C, bevorzugt 47 °C bis 170 °C, bevorzugter 60 °C bis 150 °C durchgeführt und insbesondere bei einem Druck **p_{RRB}** von 0.5 bar bis 40 bar, bevorzugt im Bereich von 0.75 bar bis 20 bar, bevorzugter im Bereich von 0.9 bar bis 10 bar, bevorzugter im Bereich von 1 bar bis 7 bar, noch bevorzugter bei 1 bar bis 5 bar, durchgeführt.

Die Reaktionskolonne **RR_{B}** umfasst in einer bevorzugten Ausführungsform mindestens einen Verdampfer, der insbesondere aus Zwischenverdampfern **VZ_{RRB}** und Sumpfverdampfern **VS_{RRB}** ausgewählt ist. Die Reaktionskolonne **RR_{B}** umfasst besonders bevorzugt mindestens einen Sumpfverdampfer **VS_{RRB}.**

Im optionalen Schritt (a2) des erfindungsgemäßen Verfahrens wird am unteren Ende der Reaktionskolonne **RR_{B}** ein Sumpfproduktstrom **S_{BS}** umfassend ROH und M_{B}OR entnommen.

Es ist bevorzugt, dass die Reaktionskolonne **RR_{B}** mindestens einen Sumpfverdampfer **VS_{RRB}** aufweist, über den Sumpfproduktstrom **S_{BS}** dann mindestens teilweise geleitet wird.

Bevorzugt weist der in Schritt (a2) entnommene Sumpfproduktstrom **S_{BS}** einen Massenanteil von M_{B}OR in ROH Bereich 1 bis 50 Gew.-%, bevorzugt 5 bis 32 Gew.-%, bevorzugter 10 bis 32 Gew.%, noch bevorzugter 15 bis 32 Gew.-% auf, jeweils bezogen auf die Gesamtmasse von **S_{BS}**.

Der Massenanteil an Restwasser in **S_{BS}** liegt dabei bevorzugt bei < 1 Gew.-%, bevorzugt < 0.5 Gew.-%, bevorzugter < 0.2 Gew.-%, bezogen auf die Gesamtmasse von **S_{BS}**.

Der Massenanteil an Edukt M_{B}OH in **S_{BS}** liegt dabei bevorzugt bei < 1 Gew.-%, bevorzugt < 0.5 Gew.-%, bevorzugter < 0.2 Gew.-%, bezogen auf die Gesamtmasse von **S_{BS}**.

In einer bevorzugten Ausführungsform des vorliegenden Verfahrens wird Schritt (a2) durchgeführt.

Dies hat den Vorteil, dass zwei verschiedene Alkalimetallalkoholate hergestellt werden können, ohne eine Querkontamination des einen Alkalimetallalkoholats mit dem anderen zu verursachen oder Produktverluste beim Umstellen des Eduktes M_{A}OH auf M_{B}OH zu erleiden.

Der optionale Schritt (a2) des erfindungsgemäßen Verfahrens wird gleichzeitig mit und räumlich getrennt von Schritt (a1) durchgeführt. Die räumliche Trennung wird durch die Durchführung der Schritte (a1) und (a2) in den beiden Reaktionskolonnen **RR_{A}** und **RR_{B}** gewährleistet.

In einer vorteilhaften Ausgestaltung der Erfindung sind die Reaktionskolonnen **RR_{A}** und **RR_{B}** in einem Kolonnenmantel untergebracht, wobei die Kolonne mindestens teilweise durch mindestens eine Trennwand unterteilt ist. Eine solche mindestens eine Trennwand aufweisende Kolonne wird erfindungsgemäß als "TRD" bezeichnet. Solche Trennwandkolonnen sind dem Fachmann bekannt und beispielsweise beschrieben in US 2,295,256, EP 0 122 367 A2, EP 0 126 288 A2, WO 2010/097318 A1 sowie von I. Dejanović, Lj. Matijašević, 2. Olujić, *Chemical Engineering and Processing* 2010, 49, 559-580. In den für das erfindungsgemäße Verfahren in Frage kommenden Trennwandkolonnen sind die Trennwände bevorzugt bis zum Boden durchgezogen und durchspannen insbesondere bevorzugt mindestens ein Viertel, bevorzugter mindestens ein Drittel, noch bevorzugter mindestens die Hälfte, noch bevorzugter mindesten zwei Drittel, noch mehr bevorzugter mindestens drei Viertel der Kolonne der Länge nach. Sie teilen die Kolonne in mindestens zwei Reaktionsräume, in denen räumlich getrennte Reaktionen stattfinden können. Die durch die mindestens eine Trennwand geschaffenen Reaktionsräume können gleich oder unterschiedlich groß sein.

In dem jeweils von der Trennwand getrennten Bereichen können in dieser Ausführungsform die Sumpfproduktströme **S_{AS}** und **S_{BS}** getrennt entnommen werden und bevorzugt über den für jeden durch die mindestens eine Reaktionswand gebildeten Reaktionsraum angebrachten Sumpfverdampfer **VS_{RRA}** bzw. **VS_{RRB}** geleitet werden , in dem durch Entfernung von ROH aus **S_{AS}** bzw. **S_{BS}** eine gewünschte Konzentration von M_{A}OR oder M_{B}OR eingestellt werden kann.

### 4.3 Schritt (b) des erfindungsgemäßen Verfahrens

Im Schritt (b) des erfindungsgemäßen Verfahrens wird der Brüdenstrom **S_{AB}**, und, wenn Schritt (a2) durchgeführt wird, der Brüdenstrom **S_{BB}**, vermischt mit **S_{AB}** oder getrennt von **S_{AB}**, in eine Rektifikationskolonne **RD_{A}** geleitet, so dass in der Rektifikationskolonne **RD_{A}** ein Gemisch **G_{A}** umfassend Wasser und ROH vorliegt.

In der optionalen Ausführung des erfindungsgemäßen Verfahrens, in der Schritt (a2) durchgeführt wird, wird der Brüdenstrom **S_{BB}** dabei bevorzugt vermischt mit **S_{AB}** und dann der erhaltene Mischbrüden in die Rektifikationskolonne **RD_{A}** geleitet.

Der Brüdenstrom **S_{AB}** und, in den Fällen, in denen der optionale Schritt (a2) durchgeführt wird, der Brüdenstrom **S_{BB}**, können in einer Ausführungsform der vorliegenden Erfindung verdichtet werden, bevor sie in die Rektifikationskolonne **RD_{A}** geleitet werden. Dies kann über einen Verdichter **VD₃₁** geschehen.

Der Fachmann kann gemäß seinem Fachwissen zwischen den Kolonnen einen oder mehrere Verdichter vorsehen. Insbesondere ist bei gasförmigen Strömen, die zwei oder mehrere Kolonnen verbinden, immer dann ein Verdichter/Verdichtungsschritt vorzusehen, wenn der Druck in der Kolonne in den ein gasförmiger Strom geführt wird, höher ist als der Druck in der Kolonne, aus der der betreffende Strom austritt.

Es versteht sich von selbst, dass auch in den Ausführungsformen, in denen der optionale Schritt (a2) durchgeführt wird, und **S_{BB}** getrennt von **S_{AB}** in die Rektifikationskolonne **RD_{A}** geleitet wird, sich **S_{AB}** und **S_{BB}** in der Rektifikationskolonne **RD_{A}** vermischen, so dass in jedem Fall nach Durchführung von Schritt (b) ein Gemisch **G_{A}** umfassend Wasser und ROH in der Rektifikationskolonne **RD_{A}** erhalten wird.

Als Rektifikationskolonne **RD_{A}** in Schritt (b) des erfindungsgemäßen Verfahrens können dem Fachmann bekannte Rektifikationskolonnen eingesetzt werden. Bevorzugt enthält die Rektifikationskolonne **RD_{A}** Einbauten. Geeignete Einbauten sind zum Beispiel Böden, unstrukturierte Packungen oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox^{®}-Sättel verwendet. Strukturierte Packungen werden zum Beispiel unter dem Handelsnamen Mellapack^{®} der Firma Sulzer vertrieben. Neben den genannten Einbauten weitere geeignete Einbauten sind dem Fachmann bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Rektifikationskolonne möglichst gering bleibt und somit die mechanische Leistung des Verdichters und die Temperatur des zu verdampfenden Alkohol/Wasser-Gemischs **G_{A}** gering bleibt.

Wenn in der Rektifikationskolonne **RD_{A}** strukturierte Packungen oder unstrukturierte Packungen enthalten sind, so können diese geteilt sein oder es kann eine durchgehende Packung vorliegen. Üblicherweise sind jedoch mindestens zwei Packungssektionen vorgesehen, eine Packungssektion oberhalb der Zulaufstelle des zu trennenden Brüdenstroms (dies ist **S_{AB}** oder, gemischt oder getrennt voneinander, **S_{AB}** und **S_{BB}**) und eine Packungssektion unterhalb der Zulaufstelle des zu trennenden Brüdenstroms. Wenn eine Packung eingesetzt wird, beispielsweise eine Füllkörperpackung, so liegen die Füllkörper üblicherweise auf einem geeigneten Trägersystem auf. Bevorzugt ist eine Ausführungsform, in der oberhalb des Zulaufs Packungen und unterhalb des Zulaufs Böden in der Rektifikationskolonne **RD_{A}** angebracht sind.

Die Rektifikationskolonne **RD_{A}** kann auch Einbauten zur Verhinderung des Tropfenmitrisses umfassen, wie sie für die Reaktionskolonne **RR_{A}** beschrieben sind.

In einer vorteilhaften Ausgestaltung der Erfindung sind die Reaktionskolonne **RR_{A}** gemäß Schritt (a1), bzw. Reaktionskolonnen **RR_{A}** und **RR_{B}** in der vorbeschriebenen bevorzugten Ausführungsform, in der Schritt (a2) durchgeführt wird, und die Rektifikationskolonne **RD_{A}** gemäß Schritt (b) zur Auftrennung des Gemischs **G_{A}** in einem Kolonnenmantel untergebracht, wobei die Kolonne mindestens teilweise durch mindestens eine Trennwand, bzw. in der vorbeschriebenen bevorzugten Ausführungsform, in der Schritt (a2) durchgeführt wird, durch mindestens zwei Trennwände, unterteilt ist, wobei die mindestens eine Trennwand bzw. die mindestens zwei Trennwände bis zum Boden der Kolonne durchgezogen sind. Wie unter Punkt 4.2 beschrieben, handelt es sich dann wieder um eine Trennwandkolonne.

In diesem Fall wird in einem Teil der TRD die Reaktion zum Rohproduktgemisch **RP_{A}** gemäß Schritt (a1) bzw. den Rohproduktgemischen **RP_{A}** und **RP_{B}** gemäß Schritten (a1) und (a2) durchgeführt, wobei der Eduktstrom **S_{AE2}** und gegebenenfalls der Eduktstrom **S_{BE2}** zwar unterhalb, aber in etwa auf der Höhe des oberen Endes der Trennwand zugegeben wird und der Eduktstrom **S_{AE1}** und gegebenenfalls der Eduktstrom **S_{BE1}** dampfförmig am unteren Ende aufgegeben wird. Das oberhalb der Zugabestelle des Eduktstroms entstehende Alkohol/Wasser-Gemisch verteilt sich dann oberhalb der Trennwand über den gesamten Kolonnenbereich, der als Verstärkungsteil der Rektifikationskolonne **RD_{A}** dient. Der zweite, durch die Trennwand abgetrennte untere Teil der Kolonne ist der Abtriebsteil der Rektifikationskolonne **RD_{A}**. Die für die Destillation notwendige Energie wird dann über einen Verdampfer am unteren Ende des zweiten durch die Trennwand abgetrennten Teils der Kolonne zugeführt, wobei dieser Verdampfer konventionell beheizt werden kann oder mit Heizdampf, zum Beispiel **H₁** oder **H₄**, beheizt werden kann. Wenn der Verdampfer konventionell beheizt wird, so kann zusätzlich ein Zwischenverdampfer vorgesehen werden, der mit Heizdampf, zum Beispiel **H₁** oder **H₄**, beheizt werden kann.

Am Ende des Schrittes (b) des erfindungsgemäßen Verfahrens wird schließlich ein Gemisch **G_{A}** umfassend Wasser und ROH in der Rektifikationskolonne **RD_{A}** erhalten. Die Zusammensetzung des Gemisches **G_{A}** ergibt sich insbesondere aus der Zusammensetzung des Brüdenstroms **S_{AB}** bzw., wenn Schritt (a2) durchgeführt wird, insbesondere anteilig aus der Zusammensetzung der beiden Brüdenströme **S_{AB}** und **S_{BB}**.

### 4.4 Schritt (c) des erfindungsgemäßen Verfahrens

Im Schritt (c) des erfindungsgemäßen Verfahrens wird das Gemisch **G_{A}** umfassend Wasser und ROH in **RD_{A}** in einen ROH umfassenden Brüdenstrom **S_{DAB}** am oberen Ende von **RD_{A}** und einen Sumpfstrom **S_{DAS}** umfassend Wasser und ROH am unteren Ende von **RD_{A}** aufgetrennt.

Der Druck **p_{A}** in **RD_{A}** kann vom Fachmann gemäß seinem Fachwissen eingestellt werden. Er liegt bevorzugt im Bereich zwischen 1 bar und 20 bar, bevorzugt 1 bar und 15 bar, bevorzugter 1 bis 10 bar.

Die Temperatur **T_{A}** in **RD_{A}** kann vom Fachmann gemäß seinem Fachwissen eingestellt werden. Die liegt bevorzugt im Bereich von 40 °C bis 220 °C, bevorzugt 60 °C bis 190 °C.

Bei der Auftrennung gemäß Schritt (c) des erfindungsgemäßen Verfahrens handelt es sich um eine dem Fachmann bekannte destillative Auftrennung des Alkohol-Wasser-Gemischs **G_{A}**.

Am Sumpf (anderes Wort für "Sumpf' ist "am unteren Ende") der Rektifikationskolonne **RD_{A}** wird ein Strom **S_{DAS}** erhalten, der Alkohol aufweist.

Am Kopf (anderes Wort für "Kopf" ist "am oberen Ende") der Rektifikationskolonne **RD_{A}** wird außerdem der Brüdenstrom **S_{DAB}** umfassend ROH erhalten. Der bevorzugte Massenanteil von ROH in diesem Brüdenstrom **S_{DAB}** ist ≥ 99 Gew.-%, bevorzugt ≥ 99.6 Gew.-%, bevorzugter ≥ 99.9 Gew.-%, jeweils bezogen auf die Gesamtmasse von **S_{DAB}**, wobei der Rest insbesondere Wasser ist.

Der Sumpfstrom **S_{DAS}** wird an einer Entnahmestelle **E_{AS}** am unteren Ende von **RD_{A}** aus **RD_{A}** geleitet und der Brüdenstrom **S_{DAB}** an einer Entnahmestelle **E_{AK}** am oberen Ende von **RD_{A}** aus **RD_{A}** geleitet.

"Entnahmestelle **E_{AK}** am oberen Ende von **RD_{A}**" bedeutet im Rahmen der vorliegenden Erfindung, dass **E_{AK}** so an der Kolonne angebracht ist, dass der Brüdenstrom **S_{DAB}** als Kopfstrom oder als Seitenabzug oberhalb der Einbauten in der Rektifikationskolonne **RD_{A}** entnommen wird.

"Entnahmestelle **E_{AS}** am unteren Ende von **RD_{A}**" bedeutet im Rahmen der vorliegenden Erfindung, dass **E_{AS}** so an der Kolonne angebracht ist, dass der Sumpfstrom **S_{DAS}** als Sumpfstrom oder als Seitenabzug unterhalb der Einbauten in der Rektifikationskolonne **RD_{A}** entnommen wird.

In einer optionalen Ausführungsform des erfindungsgemäßen Verfahrens wird an der einen Rektifikationskolonne **RD_{A}** an einer Entnahmestelle **E_{AZ}** mindestens ein weiterer, von **S_{DAS}** und **S_{DAB}** verschiedener Strom **S_{AZ}** umfassend ROH und Wasser aus **RD_{A}** geleitet, auf diesen Energie übertragen, und in **RD_{A}** rückgeleitet, wobei sich die Position der Entnahmestelle **E_{AZ}** an **RD_{A}** zwischen den beiden Entnahmestellen **E_{AS}** und **E_{AK}** befindet. Der Strom **S_{AZ}** umfasst dabei das Gemisch **G_{A}**.

In dieser optionalen Ausführungsform des Schrittes (c) des erfindungsgemäßen Verfahrens ist es möglich, dem Gemisch **G_{A}** Energie zuzuführen. In einer bevorzugten Ausführungsform geschieht dies dergestalt, dass das Gemisch **G_{A}** als Strom **S_{AZ}** abgezogen und über einen Zwischenverdampfer **VZ_{A}** geleitet wird, in welchem Energie von einem Wärmemedium, insbesondere von Heizdampf **H₁** oder **H₄** oder einem davon verschiedenen Wärmemedium **W₁**, auf **S_{AZ}** bzw. **G_{A}** übertragen wird. Diese Energieübertragung kann vorteilhafterweise vorgenommen werden, in dem das Gemisch **G_{A}** als Strom **S_{AZ}** und W, oder das Gemisch **G_{A}** als Strom **S_{AZ}** und der Heizdampf **H₁** oder **H₄** über einen Zwischenverdampfer **VZ_{A}** geleitet werden.

Mindestens ein Teil des bei der Destillation gewonnenen Alkohols ROH im Brüdenstrom **S_{DAB}** wird bevorzugt der Reaktionskolonne **RR_{A}** mindestens als Teil des Eduktstroms **S_{AE1}** und, in den Fällen, in denen im erfindungsgemäßen Verfahren Schritt (a2) durchgeführt wird, alternativ oder zusätzlich der Reaktionskolonne **RR_{B}** mindestens als Teil des Eduktstroms **S_{BE1}** zugeführt.

In Schritt (c) erfolgt die destillative Trennung des in Schritt (a1) bzw. Schritt (a1) und (a2) erhaltenen Brüden **S_{AB}** bzw. **S_{AB}** und **S_{BB}**. Diese umfassen im Wesentlichen den Alkohol ROH und Wasser. Insbesondere handelt es sich bei **S_{AB}** bzw. **S_{AB}** und **S_{BB}** um eine Wasser-Alkohol-Mischung, in dem der Massenanteil von ROH bevorzugt im Bereich > 80 Gew.-%, bevorzugter > 85, noch bevorzugter > 90 Gew.-% (bezogen auf die Gesamtmasse von **S_{AB}** bzw. **S_{AB}** und **S_{BB}**) liegt. Somit ist insbesondere auch **G_{A}** ein Alkohol-Wasser-Gemisch, in dem der Massenanteil von ROH bevorzugt im Bereich > 80 Gew.-%, bevorzugter > 95 Gew.-%, noch bevorzugter > 90 Gew.% (bezogen auf die Gesamtmasse von **G_{A}**) liegt.

Der in Schritt (c) entnommene Sumpfstrom **S_{DAS}** umfasst ROH in einem Massenanteil von insbesondere 0.005 bis 95 Gew.-%, bezogen auf die Gesamtmasse von **S_{DAS}**. Bevorzugt sind 25 bis 95 Gew.-% in den Fällen, in denen Schritt (d) durchgeführt wird, und 0.005 bis 3 Gew.-% in den Fällen, in denen Schritt (d) nicht durchgeführt wird, jeweils bezogen auf die Gesamtmasse von **S_{DAS}**. Bevorzugt umfasst **S_{DAS}** neben dem Alkohol ROH im Wesentlichen Wasser.

Im erfindungsgemäßen Verfahren wird der Alkohol ROH verbraucht, und insbesondere bei kontinuierlicher Verfahrensführung muss dieser deshalb mit frischem Alkohol ROH ersetzt werden.

Frischer Alkohol ROH wird insbesondere in mindestens eine der Kolonnen ausgewählt aus Reaktivrektifikationskolonne **RD_{A}**, Reaktionskolonne **RR_{A}** zugegeben und, wenn Schritt (a2) durchgeführt wird, alternativ oder zusätzlich in die Reaktionskolonne **RR_{B}** zugegeben und, wenn Schritt (d) durchgeführt wird, alternativ oder zusätzlich in die mindestens eine Reaktionskolonne **RD_{X}** zugegeben.

Die Zuführung des frischen Alkohols ROH erfolgt dabei insbesondere direkt als Eduktstrom **S_{AE1}** umfassend ROH in die Reaktionskolonne **RR_{A}** bzw. in den Ausführungsformen, in denen Schritt (a2) durchgeführt wird, in die Reaktionskolonnen **RR_{A}** und **RR_{B}**.

Mindestens ein Teil des an der Entnahmestelle **E_{AK}** am oberen Ende der Rektifikationskolonne **RD_{A}** entnommenen Brüdenstroms **S_{AB}** wird bevorzugt der Reaktionskolonne **RR_{A}** mindestens als Teil des Eduktstroms **S_{AE1}** und, in den Fällen, in denen im erfindungsgemäßen Verfahren Schritt (a2) durchgeführt wird, alternativ oder zusätzlich der Reaktionskolonne **RR_{B}** mindestens als Teil des Eduktstroms **S_{BE1}** zugeführt.

In einer weiteren bevorzugten Ausführungsform, in der Schritt (d) durchgeführt wird, kann der Brüdenstrom **S_{XB}** mindestens teilweise als Eduktstrom **S_{AE1}** in Schritt (a1) und gegebenenfalls als Eduktstrom **S_{BE1}** in Schritt (a2) eingesetzt werden.

In der besonders bevorzugten Ausführungsform, in der Schritt (d) durchgeführt wird, können **S_{DAB}** und **S_{XB}** mindestens teilweise als Eduktstrom **S_{AE1}** in Schritt (a1) und gegebenenfalls als Eduktstrom **S_{BE1}** in Schritt (a2) eingesetzt werden. Dann können **S_{DAB}** und **S_{XB}** getrennt voneinander der jeweiligen Reaktivrektifikationskolonne **RR_{A}** bzw. **RR_{B}** zugeführt werden oder erst miteinander vermischt und dann der jeweiligen Reaktivrektifikationskolonne **RR_{A}** bzw. **RR_{B}** zugeführt werden. Bevorzugt werden in dieser Ausführungsform **S_{DAB}** und **S_{XB}** erst miteinander vermischt und dann der jeweiligen Reaktivrektifikationskolonne **RR_{A}** bzw. **RR_{B}** zugeführt.

In dieser bevorzugten Ausführungsform ist es noch bevorzugter, wenn der frische Alkohol ROH mindestens einer der Rektifikationskolonnen **RD_{A}, RD_{X},** bevorzugt beiden Kolonnen **RD_{A}** und **RD_{X},** zugegeben wird.

Wenn der frische Alkohol ROH der Rektifikationskolonne **RD_{A}** oder **RD_{X}** zugegeben wird, wird er bevorzugt entweder im Verstärkungsteil der jeweiligen Rektifikationskolonne oder direkt am Kopf der jeweiligen Rektifikationskolonne zugeführt. Die optimale Zulaufstelle ist abhängig vom Wassergehalt des eingesetzten frischen Alkohols und andererseits von dem gewünschten Restwassergehalt im Brüdenstrom **RD_{A}** bzw. **RD_{X}**. Je höher der Wasseranteil im eingesetzten Alkohol und je höher die Reinheitsanforderung im Brüdenstrom **S_{DAB}** bzw. **S_{XB}** ist, um so günstiger ist ein Zulauf einige theoretische Böden unterhalb des Kopfes der Rektifikationskolonne **RD_{A}** bzw. **RD_{X}**. Bevorzugt sind bis zu 20 theoretische Böden unterhalb des Kopfes der Rektifikationskolonne **RD_{A}** bzw. **RD_{X}** und insbesondere 1 bis 5 theoretische Böden.

Wenn der frische Alkohol ROH der Rektifikationskolonne **RD_{A}** bzw. **RD_{B}** zugegeben wird, wird er bei Temperaturen bis hin zum Siedepunkt, bevorzugt bei Raumtemperatur, zugegeben. Auch eine gasförmige Zugabe des frischen Alkohols ROH in möglich. Hierbei kann für den frischen Alkohol ein eigener Zulauf vorgesehen sein

### 4.5 Schritt (d) des erfindungsgemäßen Verfahrens (optional)

Der Schritt (d) des erfindungsgemäßen Verfahrens wird optional durchgeführt. Im optionalen Schritt (d) des erfindungsgemäßen Verfahrens wird **S_{DAS}**, ganz oder teilweise, in mindestens einer, von **RD_{A}** verschiedenen, Rektifikationskolonne **RD_{X}** in einen Brüdenstrom **S_{XB}** umfassend ROH am oberen Ende von **RD_{X}** und einen Sumpfstrom **S_{XS}** umfassend Wasser und gegebenenfalls ROH am unteren Ende von **RD_{X}** aufgetrennt.

Dadurch liegt bei Durchführung des Schrittes (d) ein Gemisch **G_{X}** umfassend Wasser und ROH in der mindestens einen Rektifikationskolonne **RD_{X}** vor.

Der Sumpfstrom **S_{XS}** wird an einer Entnahmestelle **E_{XS}** am unteren Ende der mindestens einen Rektifikationskolonne **RD_{X}** aus dieser geleitet und der Brüdenstrom **S_{XB}** wird an einer Entnahmestelle **E_{XK}** am oberen Ende der mindestens einen Rektifikationskolonne **RD_{X}** aus dieser geleitet.

"Entnahmestelle **E_{XK}** am oberen Ende der mindestens einen Rektifikationskolonne **RD_{X}**" bedeutet im Rahmen der vorliegenden Erfindung, dass **E_{XK}** so an der mindestens einen Rektifikationskolonne **RD_{X}** angebracht ist, dass der Brüdenstrom **S_{XB}** der mindestens einen Rektifikationskolonne **RD_{X}** als Kopfstrom oder als Seitenabzug oberhalb der Einbauten entnommen wird.

"Entnahmestelle **E_{XS}** am unteren Ende der mindestens einen Rektifikationskolonne **RD_{X}**" bedeutet im Rahmen der vorliegenden Erfindung, dass **E_{XS}** so an der Kolonne angebracht ist, dass der Sumpfstrom **S_{XS}** der mindestens einen Rektifikationskolonne **RD_{X}** als Sumpfstrom oder als Seitenabzug unterhalb der Einbauten entnommen wird.

Der Druck **p_{X}** in **RD_{X}** kann vom Fachmann gemäß seinem Fachwissen eingestellt werden. Er liegt bevorzugt im Bereich zwischen 1 bar und 20 bar, bevorzugt 1 bar und 15 bar, bevorzugter 1 bar bis 10 bar.

Die Temperatur **T_{X}** in **RD_{X}** kann vom Fachmann gemäß seinem Fachwissen eingestellt werden. Die liegt bevorzugt im Bereich von 40 °C bis 220 °C, bevorzugt 60 °C bis 190 °C.

Das Merkmal "mindestens einer, von **RD_{A}** verschiedenen, Rektifikationskolonne **RD_{X}**" bedeutet, dass der optionale Schritt (d) des erfindungsgemäßen Verfahrens den Fall umfasst, dass die Auftrennung des Brüdenstrom **S_{DAS}** in nur einer Rektifikationskolonne **RD_{X}** vorgenommen wird. Es umfasst aber auch den Fall umfasst, dass die Auftrennung des Brüdenstrom **S_{DAS}** in mehr als einer Rektifikationskolonne **RD_{X}** vorgenommen wird.

In den Ausführungsform der vorliegenden Erfindung, in der mehr als eine Kolonne **RD_{X}** zur Auftrennung von **S_{DAS}** eingesetzt wird, und somit mehr als eine Kolonne **RD_{X}** auf die Kolonne **RD_{A}** folgen, wird die erste dieser Kolonnen **RD_{X}**, in die der Sumpfstrom **S_{DAS}** aus **RD_{A}** geleitet wird, als "RD_{X1}" bezeichnet, und die der Kolonne RD_{X1} folgenden Kolonne(n), in die der Sumpfstrom aus **RD_{X}**, geleitet wird, als "RDₓ₂" etc.

Somit wird jede Rektifikationskolonne **RD_{X}**, die zusätzlich zur ersten Rektifikationskolonne "**RD_{X1}**" eingesetzt wird, allgemein als "**RD_{X(n+1)}**" bezeichnet, wobei n eine ganze Zahl ist, und die Anzahl der insgesamt über die Rektifikationskolonne **RD_{X1}** hinausgehende Zahl an Rektifikationskolonnen **RD_{X}** angibt. Gleichzeitig bezeichnet die einer bestimmten Kolonne **RD_{X(n+1)}** zuzuordnende ganze Zahl "n" die Zahl an Kolonnen **RD_{X}**, die zwischen der betreffenden Kolonne **RD_{X(n+1)}** und **RD_{A}** liegt bzw. liegen.

Demnach wäre bei insgesamt zwei Kolonnen **RD_{X}** n = 1, bei drei Kolonnen **RD_{X}** n = 2 etc.

In den Ausführungsformen der vorliegenden Erfindung, in denen mehr als eine Kolonne **RD_{X}** zur Auftrennung von **S_{DAS}** eingesetzt wird, ergibt sich dann in der Rektifikationskolonnen **RD_{X}**, jeweils ein Brüdenstrom **S_{XB1}** umfassend ROH am oberen Ende von **RD_{X}**, und ein Sumpfstrom **S_{XS1}** umfassend Wasser und gegebenenfalls ROH am unteren Ende von **RD_{X1}**. In der Rektifikationskolonne **RDₓ₁** liegt das Gemisch **G_{X1}** umfassend Wasser und ROH vor.

In jeder zusätzlichen Rektifikationskolonne **RD_{X(n+1)}** ergibt sich dann dementsprechend jeweils ein Brüdenstrom **S_{XB(n+1)}** umfassend ROH am oberen Ende von **RD_{X(n+1)}** und ein Sumpfstrom **S_{XS(n+1)}** umfassend Wasser und gegebenenfalls ROH am unteren Ende von **RD_{X(n+1)}**. In der Rektifikationskolonne **RD_{X(n+1)}** liegt das Gemisch **G_{X(n+1)}** umfassend Wasser und ROH vor. "n" ist dabei eine ganze Zahl mit der vorbeschriebenen Bedeutung.

In den Ausführungsform der vorliegenden Erfindung, in der mehr als eine Kolonne **RD_{X}** zur Auftrennung von **S_{DAS}** eingesetzt wird, wird diese Auftrennung dann so vorgenommen, dass **S_{DAS}** in **RD_{X}**, in einen Brüdenstrom **S_{XB1}** umfassend ROH am oberen Ende von **RD_{X}**, und einen Sumpfstrom **S_{XS1}** umfassend Wasser und gegebenenfalls ROH am unteren Ende von **RD_{X}**, aufgetrennt wird. In jeder **RD_{X}**, folgenden Kolonne **RD_{X(n+1)}** wird dann der Sumpfstrom **S_{XSn}** der vorhergehenden Kolonne **RD_{Xn}** in einen Brüdenstrom **S_{XB(n+1)}** umfassend ROH am oberen Ende von **RD_{X(n+1)}** und einen Sumpfstrom **S_{XS(n+1)}** umfassend Wasser und gegebenenfalls ROH am unteren Ende von **RD_{X(n+1)}** aufgetrennt.

In Summe wird somit auch in dieser Ausführungsform **S_{DAS}** in einen Brüdenstrom **S_{XB}** umfassend ROH [der sich dann aus den einzelnen Brüdenströmen **S_{XB1}** und **S_{XB(n+1)}** zusammensetzt], und einen Sumpfstrom **S_{XS}** umfassend Wasser und gegebenenfalls ROH [der dann dem Sumpfstrom **S_{XS(n+1)}** der letzten Kolonne **RD_{X(n+1)}** entspricht] aufgetrennt.

Der Sumpfstrom **S_{DAS}** kann in einer Ausführungsform der vorliegenden Erfindung über eine Pumpe in die mindestens eine Rektifikationskolonne **RD_{X}** geleitet werden. Genauso kann bei mehreren Rektifikationskolonnen **RD_{X}** jeweils eine Pumpe eingesetzt werden, um den Sumpfstrom **S_{XSn}** einer Rektifikationskolonne **RD_{Xn}** in die Rektifikationskolonne **RD_{X(n+1)}** zu befördern.

Als Rektifikationskolonne **RD_{X}** geeignete Rektifikationskolonnen sind dem Fachmann bekannt. Bevorzugte Ausgestaltungen wie z.B. Einbauten etc. in **RD_{X}** sind jene in Abschnitt 4.3 für **RD_{A}** beschriebenen.

Bei der Auftrennung gemäß Schritt (d) des erfindungsgemäßen Verfahrens handelt es sich um eine dem Fachmann bekannte destillative Auftrennung des Alkohol-Wasser-Gemischs **G_{X}**, welches sich in der mindestens einen Rektifikationskolonne **RD_{X}** ergibt, wenn der Sumpfstrom **S_{DAS}** in die mindestens eine Rektifikationskolonne **RD_{X}** eingeleitet wird.

Wird nur eine Rektifikationskolonne **RD_{X}** eingesetzt, weist der in Schritt (d) an der Rektifikationskolonnen **RD_{X}** entnommene Sumpfstrom **S_{XS}** ROH in einem Massenanteil von insbesondere 0.0001 bis 3 Gew.-%, bevorzugt 0.005 bis 1 Gew.-%, jeweils bezogen auf die Gesamtmasse von **S_{XS1}** auf. Bevorzugt umfasst **S_{XS}** neben dem Alkohol ROH im Wesentlichen Wasser.

Werden mehr als eine Rektifikationskolonne **RD_{X}** eingesetzt, weist der Sumpfstrom **S_{XS(n+1)}**, der an der letzten Rektifikationskolonnen **RD_{X(n+1)}** entnommen wurde, ROH in einem Massenanteil von insbesondere 0.0001 bis 3 Gew.-%, bevorzugt 0.005 bis 1 Gew.-%, jeweils bezogen auf die Gesamtmasse von **S_{XS}**, auf. Bevorzugt umfasst **S_{XS}** neben dem Alkohol ROH im Wesentlichen Wasser.

Am Kopf (anderes Wort für "Kopf" ist "am oberen Ende") der mindestens einen Rektifikationskolonne **RD_{X}** wird außerdem der Brüdenstrom **S_{XB}** umfassend ROH erhalten. Der bevorzugte Massenanteil von ROH in diesem Brüdenstrom **S_{XB}** ist ≥ 99 Gew.-%, bevorzugt ≥ 99.6 Gew.-%, bevorzugter ≥ 99.9 Gew.-%, jeweils bezogen auf die Gesamtmasse von **S_{XB}**, wobei der Rest insbesondere Wasser ist.

In einer optionalen Ausführungsform des erfindungsgemäßen Verfahrens wird an der mindestens einen Rektifikationskolonne **RD_{X}** an einer Entnahmestelle **E_{XZ}** mindestens ein weiterer, von **S_{XS}** und **S_{XB}** verschiedener Strom **S_{XZ}** umfassend ROH und Wasser aus der mindestens einen Rektifikationskolonne **RD_{X}** geleitet, auf diesen Energie übertragen und in dieselbe mindestens eine Rektifikationskolonne **RD_{X}** rückgeleitet, wobei sich die Position der Entnahmestelle **E_{XZ}** an der mindestens einen Rektifikationskolonne **RD_{A}** zwischen den beiden Entnahmestellen **E_{XS}** und **E_{XK}** befindet. Der Strom **S_{XZ}** umfasst dabei das Gemisch **G_{X}**.

In dieser optionalen Ausführungsform des optionalen Schrittes (d) des erfindungsgemäßen Verfahrens ist es möglich, dem Gemisch **G_{X}** Energie zuzuführen. In einer bevorzugten Ausführungsform geschieht dies dergestalt, dass das Gemisch **G_{X}** als Strom **S_{XZ}** über einen Zwischenverdampfer **VZ_{X}** geleitet wird, in welchem Energie von einem Wärmemedium, insbesondere von Heizdampf **H₁** oder **H₄** oder einem davon verschiedenen Wärmemedium **W₁**, auf **S_{XZ}** bzw. **G_{X}** übertragen wird. Diese Energieübertragung kann vorteilhafterweise vorgenommen werden, in dem das Gemisch **G_{X}** als Strom **S_{XZ}** und **W,** oder das Gemisch **G_{X}** als Strom **S_{XZ}** und der Heizdampf **H₁** oder **H₄** über einen Zwischenverdampfer **VZ_{X}** geleitet werden.

Mindestens ein Teil des an der Entnahmestelle **E_{XK}** am oberen Ende der mindestens einen Rektifikationskolonne **RD_{X}** entnommenen Brüdenstroms **S_{XB}** wird bevorzugt der Reaktionskolonne **RR_{A}** mindestens als Teil des Eduktstroms **S_{AE1}** und, in den Fällen, in denen im erfindungsgemäßen Verfahren Schritt (a2) durchgeführt wird, alternativ oder zusätzlich der Reaktionskolonne **RR_{B}** mindestens als Teil des Eduktstroms **S_{BE1}** zugeführt.

Wenn sowohl **S_{DAB}** als auch **S_{XB}** mindestens als Teil des Eduktstroms **S_{AE1}** und, in den Fällen, in denen im erfindungsgemäßen Verfahren Schritt (a2) durchgeführt wird, alternativ oder zusätzlich der Reaktionskolonne **RR_{B}** mindestens als Teil des Eduktstroms **S_{BE1}** zugeführt werden, können diese vorher gemischt werden und dann als ein Eduktstrom **S_{AE1}** bzw. **S_{BE1}** in die jeweilige Reaktionskolonne **RR_{A}** bzw. **RR_{B}** eingespeist werden, oder sie werden als separate Ströme in die jeweilige Reaktionskolonne **RR_{A}** bzw. **RR_{B}** eingespeist.

### 4.6 Schritte (e) und (h) des erfindungsgemäßen Verfahrens

Im Schritt (e) wird Energie, bevorzugt Wärme, von einem Heizdampf **H₁** mit Druck **p₁** auf mindestens eine Komponente **Q,** übertragen, wodurch der Heizdampf **H₁** mindestens teilweise kondensiert und so ein Kondensat **K₁** erhalten wird.

In Schritt (h) wird Energie von einem Heizdampf **H₄** mit Druck **p₄** auf mindestens eine Komponente **Q₂** übertragen wird, wodurch insbesondere der Heizdampf **H₄** mindestens teilweise kondensiert und so ein Kondensat **K₂** erhalten wird.

Als Heizdampf **H₁** oder **H₄** im Rahmen der Erfindung kann jeder Brüdenstrom verwendet werden. Er kann im Sinne der Erfindung beispielsweise n-Butan oder Wasser umfassen. Bevorzugt handelt es sich um Wasserdampf.

"Komponente **Q₁**" und die "Komponente **Q₂**" sind erfindungsgemäß Sammelbezeichnungen für die Gemische und Rohprodukte **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}**, **G_{X}**, **S_{XB}**, **S_{XS}**.

**Q₁** ist in anderen Worten aus der Gruppe bestehend aus **S_{AE1}**, **S_{AE2}**, **RP_{A}**, **S_{AS}**, **S_{AB}**, **G_{A}**, **S_{DAS}**, **S_{DAB}** ausgewählt, und kann, wenn Schritt (a2) durchgeführt wird, alternativ oder zusätzlich aus der Gruppe bestehend aus **S_{BE1}**, **S_{BE2}**, **RP_{B}**, **S_{BS}**, **S_{BB}** ausgewählt werden, und kann außerdem, wenn Schritt (d) durchgeführt wird, alternativ oder zusätzlich aus der Gruppe bestehend aus **G_{X}**, **S_{XB}**, **S_{XS}** ausgewählt werden.

Entsprechendes gilt für **Q₂**: **Q₂** ist aus der Gruppe bestehend aus **S_{AE1}**, **S_{AE2}**, **RP_{A}**, **S_{AS}**, **S_{AB}**, **G_{A}**, **S_{DAS}**, **S_{DAB}** ausgewählt, und kann, wenn Schritt (a2) durchgeführt wird, alternativ oder zusätzlich aus der Gruppe bestehend aus **S_{BE1}**, **S_{BE2}**, **RP_{B}**, **S_{BS}**, **S_{BB}** ausgewählt werden, und kann außerdem, wenn Schritt (d) durchgeführt wird, alternativ oder zusätzlich aus der Gruppe bestehend aus **G_{X}**, **S_{XB}**, **S_{XS}** ausgewählt werden.

Dies bedeutet, dass in den Ausführungsformen des erfindungsgemäßen Verfahrens, in denen Schritt (a2) durchgeführt wird, aber nicht Schritt (d) durchgeführt wird, die Komponente **Q₁** aus **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}** ausgewählt ist,
und in den Ausführungsformen des erfindungsgemäßen Verfahrens, in denen Schritt (d) durchgeführt wird, aber nicht Schritt (a2) durchgeführt wird, die Komponente **Q₁** aus **S_{AE1}**, **S_{AE2}**, **RP_{A}**, **S_{AS}**, **S_{AB}**, **G_{A}**, **S_{DAS}**, **S_{DAB}**, **G_{X}**, **S_{XB}**, **S_{XS}** ausgewählt ist,
und in den Ausführungsformen des erfindungsgemäßen Verfahrens, in denen beide Schritte (a2) und (d) durchgeführt werden, die Komponente **Q₁** aus **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}**, **G_{X}, S_{XB}**, **S_{XS}** ausgewählt ist.

Dies bedeutet, dass in den Ausführungsformen des erfindungsgemäßen Verfahrens, in denen Schritt (a2) durchgeführt wird, aber nicht Schritt (d) durchgeführt wird, die Komponente **Q₂** aus **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}** ausgewählt ist,
und in den Ausführungsformen des erfindungsgemäßen Verfahrens, in denen Schritt (d) durchgeführt wird, aber nicht Schritt (a2) durchgeführt wird, die Komponente **Q₂** aus **S_{AE1}**, **S_{AE2}**, **RP_{A}**, **S_{AS}**, **S_{AB}**, **G_{A}**, **S_{DAS}**, **S_{DAB}**, **G_{X}**, **S_{XB}**, **S_{XS}** ausgewählt ist,
und in den Ausführungsformen des erfindungsgemäßen Verfahrens, in denen beide Schritte (a2) und (d) durchgeführt werden, die Komponente **Q₂** aus **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}**, **G_{X}**, **S_{XB}**, **S_{XS}** ausgewählt ist.

**Q₁** und **Q₂** sind gleich oder verschieden und sind bevorzugt gleich.

Wenn **Q₁** und **Q₂** gleich sind, bedeutet dies erfindungsgemäß, dass das Gemisch bzw. der Strom ausgewählt aus **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}**, **G_{X}**, **S_{XB}**, **S_{XS}**, auf den in Schritt (e) Energie von Heizdampf **H₁** übertragen wird, gleich ist mit dem Gemisch bzw. dem Strom ausgewählt aus **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}**, **G_{X}**, **S_{XB}**, **Sₓₛ**, auf den in Schritt (h) Energie von Heizdampf **H₄** übertragen wird.

Wenn **Q₁** und **Q₂** verschieden sind, bedeutet dies erfindungsgemäß, dass das Gemisch bzw. der Strom ausgewählt aus **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}**, **G_{X}**, **S_{XB}**, **Sₓₛ**, auf den in Schritt (e) Energie von Heizdampf **H₁** übertragen wird, verschieden ist von dem Gemisch bzw. dem Strom ausgewählt aus **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}**, **G_{X}**, **S_{XB}**, **Sₓₛ**, auf den in Schritt (h) Energie von Heizdampf **H₄** übertragen wird.

In den Ausführungsformen der vorliegenden Erfindung, in denen mehr als eine Kolonne **RD_{X}** zur Auftrennung von **S_{DAS}** eingesetzt wird, bezeichnet "**S_{XS}**" die Gruppe bestehend aus dem Sumpfstrom **Sₓₛ**, und den n Sumpfströmen **S_{XS(n+1)}**, wobei "**S_{XS(n+1)}**" den jeweiligen Sumpfstrom der Rektifikationskolonne **RDₓ₍ₙ₊₁₎** bezeichnet.

In den Ausführungsformen der vorliegenden Erfindung, in denen mehr als eine Kolonne **RD_{X}** zur Auftrennung von **S_{DAS}** eingesetzt wird, bezeichnet "**S_{XB}**" die Gruppe bestehend aus dem Brüdenstrom **S_{XB1}** und den n Brüdenströmen **S_{XB(n+1)}**, wobei "**S_{XB(n+1)}**" den jeweiligen Brüdenstrom der Rektifikationskolonne **RDₓ₍ₙ₊₁₎** bezeichnet.

In den Ausführungsformen der vorliegenden Erfindung, in denen mehr als eine Kolonne **RD_{X}** zur Auftrennung von **S_{DAS}** eingesetzt wird, bezeichnet "**G_{X}**" die Gruppe bestehend aus dem Gemisch **G_{X1}** und den n Gemischen **G_{X(n+1)}**, wobei "**G_{X(n+1)}**" das jeweilige Gemisch **G_{X}** in der Rektifikationskolonne **RDₓ₍ₙ₊₁₎** bezeichnet.

Die mindestens eine Komponente **Q₁** ist in einer bevorzugten Ausführungsform der vorliegenden Erfindung aus der Gruppe bestehend aus **S_{AE1}**, **S_{AE2}**, **RP_{A}**, **S_{AS}**, **G_{A}**, **S_{DAS}** ausgewählt,
wobei, wenn Schritt (a2) durchgeführt wird, die mindestens eine Komponente **Q₁** alternativ oder zusätzlich aus **S_{BE1}**, **S_{BE2}**, **RP_{B}**, **S_{BS}** ausgewählt werden kann,
und wobei, wenn Schritt (d) durchgeführt wird, die mindestens eine Komponente **Q₁** alternativ oder zusätzlich aus **G_{X}**, **S_{XS}** ausgewählt werden kann.

Die mindestens eine Komponente **Q₁** ist in einer bevorzugteren Ausführungsform der vorliegenden Erfindung aus der Gruppe bestehend aus **RP_{A}**, **S_{AS}**, **G_{A}**, **S_{DAS}** ausgewählt,
wobei, wenn Schritt (a2) durchgeführt wird, die mindestens eine Komponente **Q₁** alternativ oder zusätzlich aus **RP_{B}**, **S_{BS}** ausgewählt werden kann,
und wobei, wenn Schritt (d) durchgeführt wird, die mindestens eine Komponente **Q₁** alternativ oder zusätzlich aus **G_{X}**, **S_{XS}** ausgewählt werden kann.

Die mindestens eine Komponente **Q₁** ist in einer noch bevorzugteren Ausführungsform der vorliegenden Erfindung aus der Gruppe bestehend aus **G_{A}**, **S_{DAS}** ausgewählt,
und wobei, wenn Schritt (d) durchgeführt wird, die mindestens eine Komponente **Q₁** alternativ oder zusätzlich aus **G_{X}**, **S_{XS}** ausgewählt werden kann.

Die mindestens eine Komponente **Q₁** ist in einer noch mehr bevorzugteren Ausführungsform der vorliegenden Erfindung aus der Gruppe bestehend aus **G_{A}**, **S_{DAS}** ausgewählt.

Die mindestens eine Komponente **Q₂** ist in einer bevorzugten Ausführungsform der vorliegenden Erfindung aus der Gruppe bestehend aus **S_{AE1}**, **S_{AE2}**, **RP_{A}**, **S_{AS}**, **G_{A}**, **S_{DAS}** ausgewählt,
wobei, wenn Schritt (a2) durchgeführt wird, die mindestens eine Komponente **Q₂** alternativ oder zusätzlich aus **S_{BE1}**, **S_{BE2}**, **RP_{B}**, **S_{BS}** ausgewählt werden kann,
und wobei, wenn Schritt (d) durchgeführt wird, die mindestens eine Komponente **Q₂** alternativ oder zusätzlich aus **G_{X}**, **S_{XS}** ausgewählt werden kann.

Die mindestens eine Komponente **Q₂** ist in einer bevorzugteren Ausführungsform der vorliegenden Erfindung aus der Gruppe bestehend aus **RP_{A}**, **S_{AS}**, **G_{A}**, **S_{DAS}** ausgewählt,
wobei, wenn Schritt (a2) durchgeführt wird, die mindestens eine Komponente **Q₂** alternativ oder zusätzlich aus **RP_{B}**, **S_{BS}** ausgewählt werden kann,
und wobei, wenn Schritt (d) durchgeführt wird, die mindestens eine Komponente **Q₂** alternativ oder zusätzlich aus **G_{X}**, **S_{XS}** ausgewählt werden kann.

Die mindestens eine Komponente **Q₂** ist in einer noch bevorzugten Ausführungsform der vorliegenden Erfindung aus der Gruppe bestehend aus **G_{A}**, **S_{DAS}** ausgewählt,
und wobei, wenn Schritt (d) durchgeführt wird, die mindestens eine Komponente **Q₂** alternativ oder zusätzlich aus **G_{X}**, **S_{XS}** ausgewählt werden kann.

Die mindestens eine Komponente **Q₂** ist in einer noch mehr bevorzugteren Ausführungsform der vorliegenden Erfindung aus der Gruppe bestehend aus **G_{A}**, **S_{DAS}** ausgewählt.

Die Energie des Heizdampfes **H₁** bzw. **H₄** kann in Schritt (e) bzw. Schritt (h) des erfindungsgemäßen Verfahrens mit dem Fachmann bekannten Verfahren auf die mindestens eine Komponente **Q₁** bzw. **Q₂** übertragen werden. Dazu wird insbesondere ein Wärmeübertrager **WT** eingesetzt. Als Wärmeübertrager **WT** (auch als "Wärmetauscher" bezeichnet) können sämtliche dem Fachmann zu diesem Zweck geläufigen Wärmeübertrager genutzt werden. Bevorzugt handelt es sich dabei um einen Verdampfer, bevorzugter ausgewählt aus Sumpfverdampfer VS, Zwischenverdampfer **VZ.**

Die Energieübertragung vom Heizdampf **H₁** bzw. **H₄** auf die mindestens eine Komponente **Q₁** bzw. **Q₂** erfolgt erfindungsgemäß insbesondere dadurch, dass vom Heizdampf **H₁** bzw. **H₄** Wärme auf die mindestens einen Komponente **Q₁** bzw. **Q₂** übertragen wird, der Heizdampf **H₁** bzw. **H₄** also die mindestens einen Komponente **Q₁** bzw. **Q₂** beheizt.

Die Energieübertragung vom Heizdampf **H₁** auf die mindestens eine Komponente **Q₁** erfolgt dabei insbesondere direkt oder indirekt.

Die Energieübertragung vom Heizdampf **H₄** auf die mindestens eine Komponente **Q₂** erfolgt dabei insbesondere direkt oder indirekt.

"Direkt" bedeutet im Falle von **H₁** und **Q₁**, dass **H₁** mit der mindestens einer Komponente **Q₁** kontaktiert wird, so dass Energie, bevorzugt Wärme, von **H,** auf die mindestens eine Komponente **Q,** übertragen wird. Die Kontaktierung findet dabei bevorzugt in einem Wärmeübertrager **WT,** noch bevorzugter in einem Verdampfer statt.

Im Falle von **H₄** und **Q₂** bedeutet "direkt", dass **H₄** mit der mindestens einer Komponente **Q₂** kontaktiert wird, so dass Energie, bevorzugt Wärme, von **H₄** auf die mindestens eine Komponente **Q₂** übertragen wird. Die Kontaktierung findet dabei bevorzugt in einem Wärmeübertrager WT, noch bevorzugter in einem Verdampfer statt.

Es versteht sich von selbst, dass sich bei der "direkten" als auch bei der "indirekten" Energieübertragung **H,** und **Q₁** bzw. **H₄** und **Q₂** bzw. H, oder **H₄** und **W₁** nicht mischen, da dann kein Kondensat **K₁** bzw. **K₂** erhalten würde. Die Kontaktierung ohne Vermischung wird nach dem Fachmann bekannten Verfahren erreicht, zum Beispiel über die Kontaktierung über eine Trennwand aus Metall, Kunststoff etc. insbesondere im Wärmeübertrager **WT.**

Im Falle, dass es sich bei **Q₁** oder **Q₂** um **S_{AE1}** handelt, wird die direkte Energieübertragung von **H₁** bzw. **H₄** auf **S_{AE1}** insbesondere dadurch durchgeführt, dass **S_{AE1}** und **H₁** bzw. **H₄** durch einen Wärmeübertrager **WT** (z.B. <WA4> in Abbildungen 1 bis 4) geleitet werden, bevor **S_{AE1}** in die Reaktionskolonne **RR_{A}** geleitet wird, so dass **S_{AE1}** den Heizdampf **H₁** bzw. den Heizdampf **H₄** im Wärmeübertrager **WT** kontaktiert und so Energie von **H₁** bzw. **H₄** auf **S_{AE1}** übertragen wird.

Im Falle, dass es sich bei **Q₁** oder **Q₂** um **S_{AE2}** handelt, wird die direkte Energieübertragung von **H₁** bzw. **H₄** auf **S_{AE2}** insbesondere dadurch durchgeführt, dass **S_{AE2}** und **H₁** bzw. **H₄** durch einen Wärmeübertrager **WT** (z.B. <WA5> in Abbildungen 1 bis 4) geleitet werden, bevor **S_{AE2}** in die Reaktionskolonne **RR_{A}** geleitet wird, so dass **S_{AE2}** den Heizdampf **H₁** bzw. den Heizdampf **H₄** im Wärmeübertrager **WT** kontaktiert und so Energie von **H₁** bzw. **H₄** auf **S_{AE2}** übertragen wird.

Im Falle, dass es sich bei **Q₁** oder **Q₂** um **RP_{A}** handelt, wird die direkte Energieübertragung von **H₁** bzw. **H₄** auf **RP_{A}** insbesondere dadurch durchgeführt, dass **RP_{A}** als Strom **S_{RRAZ}** aus der Kolonne **RR_{A}** geleitet wird und **S_{RRAZ}** und **H₁** bzw. **H₄** über einen Wärmeübertrager **WT,** bevorzugt einem Zwischenverdampfer **VZ_{RRA}** (z.B. <WA3> in Abbildungen 1 bis 4), an der Reaktionskolonne **RR_{A}** geleitet werden, so dass **S_{RRAZ}** den Heizdampf **H₁** bzw. den Heizdampf **H₄** im Wärmeübertrager **WT** kontaktiert und so Energie von **H₁** bzw. **H₄** auf **S_{RRAZ}** übertragen wird. Danach wird **S_{RRAZ}** zum übrigen **RP_{A}** in **RR_{A}** zurückgeführt.

Im Falle, dass es sich bei **Q₁** oder **Q₂** um **S_{AS}** handelt, wird die direkte Energieübertragung von **H₁** bzw. **H₄** auf **S_{AS}** insbesondere dadurch durchgeführt, dass **S_{AS}** und **H₁** bzw. **H₄** durch einen Wärmeübertrager **WT,** bevorzugt einem Sumpfverdampfer **VS_{RRA}** (z.B. <WA1> in Abbildungen 1 bis 4), an der Reaktionskolonne **RR_{A}** geleitet werden, so dass **S_{AS}** den Heizdampf **H₁** bzw. den Heizdampf **H₄** im Wärmeübertrager **WT** kontaktiert und so Energie von **H₁** bzw. **H₄** auf **S_{AS}** übertragen wird.

Im Falle, dass es sich bei **Q₁** oder **Q₂** um **S_{AB}** handelt, wird die direkte Energieübertragung von **H₁** bzw. **H₄** auf **S_{AB}** insbesondere dadurch durchgeführt, dass **S_{AB}** und **H₁** bzw. **H₄** durch einen Wärmeübertrager **WT** (z.B. <WA6> in Abbildungen 1 bis 4) geleitet werden, so dass **S_{AB}** den Heizdampf **H₁** bzw. den Heizdampf **H₄** im Wärmeübertrager **WT** kontaktiert und so Energie von **H₁** bzw. **H₄** auf **S_{AB}** übertragen wird.

Im Falle, dass es sich bei **Q₁** oder **Q₂** um **G_{A}** handelt, wird die direkte Energieübertragung von **H₁** bzw. **H₄** auf **G_{A}** insbesondere dadurch durchgeführt, dass **G_{A}** als Strom **S_{AZ}** aus der Kolonne **RR_{A}** geleitet wird und **S_{AZ}** und **H₁** bzw. **H₄** über einen Wärmeübertrager **WT,** bevorzugt einem Zwischenverdampfer **VZ_{A}** (z.B. <W9> in Abbildungen 1 bis 4), an der Rektifikationskolonne **RD_{A}** geleitet werden, so dass **S_{AZ}** den Heizdampf **H₁** bzw. den Heizdampf **H₄** im Wärmeübertrager **WT** kontaktiert und so Energie von **H₁** bzw. **H₄** auf **S_{AZ}** übertragen wird. Danach wird **S_{AZ}** zum übrigen **G_{A}** in **RD_{A}** zurückgeführt.

Im Falle, dass es sich bei **Q₁** oder **Q₂** um **S_{DAS}** handelt, wird die direkte Energieübertragung von **H₁** bzw. **H₄** auf **S_{DAS}** insbesondere dadurch durchgeführt, dass **S_{DAS}** und **H₁** bzw. **H₄** durch einen

Wärmeübertrager **WT,** bevorzugt einem Sumpfverdampfer **VS_{A}** (z.B. <W7> in Abbildungen 1 bis 4), an der Rektifikationskolonne **RD_{A}** geleitet werden, so dass **S_{DAS}** den Heizdampf **H₁** bzw. den Heizdampf **H₄** im Wärmeübertrager **WT** kontaktiert und so Energie von **H₁** bzw. **H₄** auf **S_{DAS}** übertragen wird.

Im Falle, dass es sich bei **Q₁** oder **Q₂** um **S_{DAB}** handelt, wird die direkte Energieübertragung von **H₁** bzw. **H₄** auf **S_{DAB}** insbesondere dadurch durchgeführt, dass **S_{DAB}** und **H₁** bzw. **H₄** durch einen Wärmeübertrager **WT** geleitet werden (nicht in den Abbildungen gezeigt), so dass **S_{DAB}** den Heizdampf **H₁** bzw. den Heizdampf **H₄** im Wärmeübertrager **WT** kontaktiert und so Energie von **H₁** bzw. **H₄** auf **S_{DAB}** übertragen wird.

Im Falle, dass es sich bei **Q₁** oder **Q₂** um **S_{BE1}** handelt, wird die direkte Energieübertragung von **H₁** bzw. **H₄** auf **S_{BE1}** insbesondere dadurch durchgeführt, dass **S_{BE1}** und **H,** bzw. **H₄** durch einen Wärmeübertrager **WT** (z.B. <WB4> in Abbildung 4) geleitet werden, bevor **S_{BE1}** in die Reaktionskolonne **RR_{B}** geleitet wird, so dass **S_{BE1}** den Heizdampf **H₁** bzw. den Heizdampf **H₄** im Wärmeübertrager **WT** kontaktiert und so Energie von **H₁** bzw. **H₄** auf **S_{BE1}** übertragen wird.

Im Falle, dass es sich bei **Q₁** oder **Q₂** um **S_{BE2}** handelt, wird die direkte Energieübertragung von **H₁** bzw. **H₄** auf **S_{BE2}** insbesondere dadurch durchgeführt, dass **S_{BE2}** und **H,** bzw. **H₄** durch einen Wärmeübertrager **WT** (z.B. <WBA5> in Abbildung 4) geleitet werden, bevor **S_{BE2}** in die Reaktionskolonne **RR_{B}** geleitet wird, so dass **S_{BE2}** den Heizdampf **H₁** bzw. den Heizdampf **H₄** im Wärmeübertrager **WT** kontaktiert und so Energie von **H₁** bzw. **H₄** auf **S_{BE2}** übertragen wird.

Im Falle, dass es sich bei **Q₁** oder **Q₂** um **RP_{B}** handelt, wird die direkte Energieübertragung von **H₁** bzw. **H₄** auf **RP_{B}** insbesondere dadurch durchgeführt, dass **RP_{B}** als Strom **S_{RRBZ}** aus der Kolonne **RR_{B}** geleitet wird und **S_{RRBZ}** und **H₁** bzw. **H₄** über einen Wärmeübertrager **WT,** bevorzugt einem Zwischenverdampfer **VZ_{RRB}** (z.B. <WB3> in Abbildung 4), an der Reaktionskolonne **RR_{B}** geleitet werden, so dass **S_{RRBZ}** den Heizdampf **H₁** bzw. den Heizdampf **H₄** im Wärmeübertrager **WT** kontaktiert und so Energie von **H₁** bzw. **H₄** auf **S_{RRBZ}** übertragen wird. Danach wird **S_{RRBZ}** zum übrigen **RP_{B}** in **RR_{B}** zurückgeführt.

Im Falle, dass es sich bei **Q₁** oder **Q₂** um **S_{BS}** handelt, wird die direkte Energieübertragung von **H₁** bzw. **H₄** auf **S_{BS}** insbesondere dadurch durchgeführt, dass **S_{BS}** und **H₁** bzw. **H₄** durch einen Wärmeübertrager **WT,** bevorzugt einem Sumpfverdampfer **VS_{RRB}** (z.B. <WB1> in Abbildung 4), an der Reaktionskolonne **RR_{B}** geleitet werden, so dass **S_{BS}** den Heizdampf **H₁** bzw. den Heizdampf **H₄** im Wärmeübertrager **WT** kontaktiert und so Energie von **H₁** bzw. **H₄** auf **S_{BS}** übertragen wird.

Im Falle, dass es sich bei **Q₁** oder **Q₂** um **S_{BB}** handelt, wird die direkte Energieübertragung von **H₁** bzw. **H₄** auf **S_{BB}** insbesondere dadurch durchgeführt, dass **S_{BB}** und **H₁** bzw. **H₄** durch einen Wärmeübertrager **WT** (z.B. <WB6> in Abbildung 4) geleitet werden, so dass **S_{BB}** den Heizdampf **H₁** bzw. den Heizdampf **H₄** im Wärmeübertrager **WT** kontaktiert und so Energie von **H₁** bzw. **H₄** auf **S_{BB}** übertragen wird.

Im Falle, dass es sich bei **Q₁** oder **Q₂** um **G_{X}** handelt, wird die direkte Energieübertragung von **H₁** bzw. **H₄** auf **G_{X}** insbesondere dadurch durchgeführt, dass **G_{X}** als Strom **S_{XZ}** aus der Kolonne **RD_{X}** geleitet wird und **S_{XZ}** und **H₁** bzw. **H₄** über einen Wärmeübertrager **WT,** bevorzugt einem Zwischenverdampfer **VZ_{X}** (z.B. <W13> in Abbildung 4), an der Rektifikationskolonne **RD_{X}** geleitet werden, so dass **S_{XZ}** den Heizdampf **H₁** bzw. den Heizdampf **H₄** im Wärmeübertrager **WT** kontaktiert und so Energie von **H₁** bzw. **H₄** auf **S_{XZ}** übertragen wird. Danach wird **S_{XZ}** zum übrigen **G_{X}** in **RD_{X}** zurückgeführt.

Im Falle, dass es sich bei **Q₁** oder **Q₂** um **S_{XS}** handelt, wird die direkte Energieübertragung von **H₁** bzw. **H₄** auf **S_{XS}** insbesondere dadurch durchgeführt, dass **S_{XS}** und **H₁** bzw. **H₄** durch einen Wärmeübertrager WT, bevorzugt einem Sumpfverdampfer **VS_{X}** (z.B. <W11> in Abbildung 4), an der Rektifikationskolonne **RD_{X}** geleitet werden, so dass **S_{XS}** den Heizdampf **H₁** bzw. den Heizdampf **H₄** im Wärmeübertrager WT kontaktiert und so Energie von **H₁** bzw. **H₄** auf **S_{XS}** übertragen wird.

Im Falle, dass es sich bei **Q₁** oder **Q₂** um **S_{XB}** handelt, wird die direkte Energieübertragung von **H₁** bzw. **H₄** auf **S_{XB}** insbesondere dadurch durchgeführt, dass **S_{XB}** und **H₁** bzw. **H₄** durch einen Wärmeübertrager **WT** geleitet werden (nicht in den Abbildungen gezeigt), so dass **S_{XB}** den Heizdampf **H₁** bzw. den Heizdampf **H₄** im Wärmeübertrager **WT** kontaktiert und so Energie von **H₁** bzw. **H₄** auf **S_{XB}** übertragen wird.

"Indirekt" bedeutet im Falle von **H₁** und **Q₁**, dass Energie von Heizdampf **H₁** auf einen Wärmeträger **W,** übertragen wird, insbesondere **H,** mit einem Wärmeträger **W,** kontaktiert wird, so dass Energie, bevorzugt Wärme, von **H₁** auf **W₁** übertragen wird, und im Anschluss daran die Energie von **W,** auf die mindestens eine Komponente **Q,** übertragen wird. Die Übertragung der Energie von **W₁** auf **Q₁** kann dann ihrerseits direkt oder indirekt stattfinden, das heißt in dem **W,** mit der mindestens einen Komponente **Q₁** kontaktiert wird, so dass Energie, bevorzugt Wärme, von **W,** auf die mindestens eine Komponente **Q₁** übergeht ("direkt") oder dass die Energie von **W,** erst auf einen oder mehrere weitere Wärmeträger **W₂**, **W₃**, **W₄** etc. übertragen wird und der letzte dieser Wärmeträger, bezeichnet als "**W_{Y}**", mit der mindestens einen Komponente **Q₁** kontaktiert wird, so dass Energie, bevorzugt Wärme, von **W_{Y}** auf die mindestens eine Komponente **Q,** übergeht. Die beschriebenen Kontaktierungen findet dabei jeweils bevorzugt in einem Wärmeübertrager **WT,** noch bevorzugter in einem Verdampfer statt.

Im Falle der indirekten Energieübertragung von **H₁** auf die mindestens eine Komponente **Q₁** ist es aber bevorzugt, dass **H,** mit einem Wärmeträger **W,** kontaktiert wird, so dass Energie, bevorzugt Wärme, von **H₁** auf **W₁** übergeht, und im Anschluss daran die Energie von **W₁** auf **Q₁** durch Kontaktierung von **W,** mit der mindestens einen Komponente **Q₁** in einem Wärmeübertrager **WT** übertragen wird.

Die Übertragung der Energie von **W,** auf **Q₁** kann in der Ausführungsform, in der es sich bei **W,** um einen Strom handelt, der im Wesentlichen Wasser und/oder den Alkohol ROH umfasst, auch so vorgenommen werden, dass **W₁** mit der mindestens einen Komponente **Q₁** gemischt wird, nachdem Energie von Heizdampf **H₁** auf **W₁**, bevorzugt in einem Wärmeübertrager **WT,** übertragen wurde. Diese Ausführungsform ist besonders dann vorteilhaft, wenn die mindestens eine Komponente **Q₁** aus der Gruppe bestehend aus **RP_{A}**, **G_{A}**, **RP_{B}**, **G_{X}**, bevorzugt aus **G_{A}**, **G_{X}**, ausgewählt ist.

"Strom, der im Wesentlichen Wasser und/oder Alkohol ROH" umfasst, bedeutet insbesondere, dass in dem Strom der Massenanteil von Alkohol ROH und Wasser ≥ 90 Gew.-%, bevorzugt ≥ 96 Gew.-%, ≥ 99 Gew.-% ist. Noch bevorzugter bedeutet dies, dass das Verhältnis der Masse an vom Strom umfassten Wasser zum vom Strom umfassten Alkohol ROH kleiner 1:1, bevorzugt 1:5, bevorzugter 1:9, bevorzugter 1:49, noch bevorzugter 1:99, noch bevorzugter 1:999ist.

"Indirekt" bedeutet im Falle von **H₄** und **Q₂**, dass Energie von Heizdampf **H₄** auf einen Wärmeträger **W,** übertragen wird, insbesondere **H₄** mit einem Wärmeträger **W,** kontaktiert wird, so dass Energie, bevorzugt Wärme, von **H₄** auf **W₁** übertragen wird, und im Anschluss daran die Energie von **W,** auf die mindestens eine Komponente **Q₂** übertragen wird. Die Übertragung der Energie von **W,** auf **Q₂** kann dann ihrerseits direkt oder indirekt stattfinden, das heißt in dem **W,** mit der mindestens einen Komponente **Q₂** kontaktiert wird, so dass Energie, bevorzugt Wärme, von **W,** auf die mindestens eine Komponente **Q₂** übergeht ("direkt") oder dass die Energie von **W,** erst auf einen oder mehrere weitere Wärmeträger **W₂**, **W₃**, **W₄** etc. übertragen wird und der letzte dieser Wärmeträger, bezeichnet als "**W_{Y}**", mit der mindestens einen Komponente **Q₂** kontaktiert wird, so dass Energie, bevorzugt Wärme, von **W_{Y}** auf die mindestens eine Komponente **Q₂** übergeht Die beschriebenen Kontaktierungen findet dabei jeweils bevorzugt in einem Wärmeübertrager, noch bevorzugter in einem Verdampfer statt.

Im Falle der indirekten Energieübertragung von **H₄** auf die mindestens eine Komponente **Q₂** ist es aber bevorzugt, dass **H₄** mit einem Wärmeträger **W,** kontaktiert wird, so dass Energie, bevorzugt Wärme, von **H₄** auf **W₁** übergeht, und im Anschluss daran die Energie von **W,** auf **Q₂** durch Kontaktierung von **W,** mit der mindestens einen Komponente **Q₂** in einem Wärmeübertrager **WT** übertragen wird.

In der Ausführungsform, in der es sich bei **W,** um einen Strom handelt, der im Wesentlichen Wasser und/oder den Alkohol ROH umfasst, wird die indirekte Übertragung von Energie von **W,** auf die mindestens einen Komponente **Q₂** bevorzugt so vorgenommen, dass **W,** mit der mindestens einen Komponente **Q₂** gemischt wird, nachdem Energie von Heizdampf **H₁** auf **W₁**, bevorzugt in einem Wärmeübertrager **WT,** übertragen wurde. Diese Ausführungsform ist besonders dann vorteilhaft, wenn die mindestens eine Komponente **Q₂** aus der Gruppe bestehend aus **RP_{A}**, **G_{A}**, **RP_{B}**, **G_{X}**, bevorzugt aus **G_{A}**, **G_{X}**, ausgewählt ist.

Die Mischung von **W,** mit der mindestens einen Komponente **Q₁** oder der mindestens einen Komponente **Q₂** wird insbesondere dadurch vorgenommen, dass **W₁**, zum Beispiel wenn es sich bei der Komponente **Q₁** bzw. **Q₂** um einen Strom **S_{AE1}**, **S_{AE2}**, **S_{AS}**, **S_{AB}**, **S_{DAS}**, **S_{DAB}**, **S_{BE1}**, **S_{BE2}**, **S_{BS}**, **S_{BB}**, **S_{XB}**, **S_{XS}** handelt, dem jeweiligen Strom in der Leitung zugeleitet wird. Wenn es sich bei der Komponente **Q₁** bzw. **Q₂** um eines der Gemische **RP_{A}**, **G_{A}**, **RP_{B}**, **G_{X}** handelt, kann **W₁** vorteilhafterweise in die Kolonne geleitet werden, in der sich das betreffende Gemisch befindet, im Falle von **RP_{A}** also in **RR_{A}**, im Falle von **RP_{B}** also in **RR_{B}**, im Falle von **G_{A}** also in **RD_{A}** und im Falle von **G_{X}** also in **RD_{X}**.

Erfindungsgemäß handelt es sich bei **W,** und allen gegebenenfalls bei der indirekten Energieübertragung eingesetzten Wärmeträger **W₂**, **W₃**, **W₄** etc. um keinen von **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}**, **G_{X}**, **S_{XB}**, **S_{XS}**.

Dies umfasst erfindungsgemäß auch den Fall, dass **W₁**, **W₂**, **W₃**, **W₄** etc. die gleichen Stoffe umfasst wie einer von **S_{AE1}**, **S_{AE2}**, **RP_{A}**, **S_{AS}**, **S_{AB}**, **G_{A}**, **S_{DAS}**, **S_{DAB}**, **S_{BE1}**, **S_{BE2}**, **RP_{B}**, **S_{BS}**, **S_{BB}**, **G_{X}**, **S_{XB}**, **S_{XS}**, und sich nur in den Massenanteilen der Stoffe von dem Strom oder dem Gemisch unterscheidet.

Als Wärmeträger **W,** bzw. gegebenenfalls daneben weiter genutzte Wärmeträger **W₂**, **W₃**, **W₄** etc. kann jeder dem Fachmann bekannte Wärmeträger genutzt werden. Bevorzugt sind sie ausgewählt aus der Gruppe bestehend aus Wasser; Alkohole, bevorzugt der Alkohol ROH; Alkohol-Wasser-Lösungen, bevorzugt ROH-Wasser-Lösungen; Salz-Wasser-Lösungen, wozu auch ionische Flüssigkeiten, wie zum Beispiel LiBr-Lösungen, Dialkylimidazoliumsalze wie insbesondere Dialkylimidazoliumdialkylphosphate, gehören; Mineralöle, wie zum Beispiel Dieselöle; Thermalöle wie zum Beispiel Silikonöle; biologische Öle wie zum Beispiel Limonen; aromatische Kohlenwasserstoffe wie zum Beispiel Dibenzyltoluol. Am bevorzugtesten wird als Wärmeträger, insbesondere als Wärmeträger **W,** Wasser, Alkohol oder Alkohol-Wasser-Lösungen eingesetzt, wobei es sich bei dem Alkohol bevorzugt um den Alkohol ROH handelt, wobei der Alkohol der gleiche ist wie jener, der auch in Schritt (a1) und gegebenenfalls in Schritt (a2) eingesetzt wird.

Salz-Wasser-Lösungen, die verwendet werden können, sind beispielsweise auch in der DE 10 2005 028 451 A1 und der WO 2006/134015 A1 beschrieben.

Diese Ausführungsform, in der Energie indirekt übertragen wird, ist demnach insbesondere vorteilhaft, wenn frischer Alkohol ROH dem System zugeführt werden soll.

Im Kontext der Erfindung bedeutet die Angabe "bar" immer "bar absolut" (= "bar abs.").

**H,** weist insbesondere eine Temperatur **T₁** von 101 °C bis 270 °C, vorzugsweise von 105 °C bis 250 °C, bevorzugt 130 °C bis 200 °C auf. **H,** weist vorzugsweise einen Druck von 1 bis 55 bar, bevorzugt von 2 bis 5 bar, bevorzugter 4 bar auf. Als Heizdampf **H₁** kann auch ein Dampf mit einem Druck von > 30 bar eingesetzt werden.

Der Heizdampf **H₁** kondensiert bei der Übertragung der Energie mindestens teilweise, wodurch vorzugsweise ein Kondensat **K₁** bei einem Druck von 1 bis 55 bar, vorzugsweise 2 bis 5 bar, besonders bevorzugt 4 bar und einer Temperatur von 101 °C bis 270 °C, vorzugsweise von 105 °C bis 250 °C, bevorzugt 110 °C bis 200 °C anfällt.

Das Heizdampfkondensat **K₁** wird bevorzugt in einem Kondensatbehälter gesammelt. Dazu können sämtliche dem Fachmann bekannten Behälter genutzt werden, z.B. aus Stahl.

### 4.7 Schritt (f) des erfindungsgemäßen Verfahrens

Im Schritt (f) des erfindungsgemäßen Verfahrens wird **K₁** mindestens teilweise zu einem Heizdampf **H₂** mit Druck **p₂** und Temperatur **T₂** entspannt wird, wobei **p₂** < **p₁**.

Die Entspannung des Heizdampfkondensats **K₁** wird vorzugsweise so durchgeführt, dass **K₁** in einen Kondensatbehälter geleitet wird, in dem das Heißkondensat vom Dampf getrennt werden kann. In einer bevorzugten Ausführungsform des erfindungsgemäßen Verfahrens wird **K₁** zur Beheizung von Strömen und/oder Gemischen im Verfahren genutzt, bevor es zu einem Heizdampf **H₂** entspannt wird. Genauso kann das verbleibende Kondensat, welches nach Entspannung auf **p₂** im flüssigen Zustand übrig bleibt, zur Beheizung von Strömen und/oder Gemischen im Verfahren genutzt werden.

In dem Kondensatbehälter liegt vorzugsweise ein geringerer Druck vor als im Wärmeübertrager (in dem **H,** mind. teilweise kondensiert wird) auf der Heizdampfseite. Aufgrund des geringeren Drucks kann ein Teil des Heißkondensats **K₁** verdampfen, wodurch der gesamte Dampf, d.h. der nicht kondensierte Anteil des Heizdampfes und das im Kondensatbehälter durch Druckentspannung verdampfte Heißkondensat, im Kondensatbehälter als Heizdampf **H₂** anfällt. Dieser Heizdampf **H₂** ist insbesondere Niederdruckdampf und weist vorzugsweise einen Druck **p₂** auf, für den gilt, dass der Quotient **p₂**/**p₁** (bar/bar) im Bereich 0.001 bis 0.999, bevorzugt 0.01 bis 0.9, bevorzugter 0.05 bis 0.8, bevorzugter 0.1 bis 0.7, bevorzugter 0.15 bis 0.6, bevorzugter 0.2 bis 0.5 beträgt.

In einer anderen bevorzugten Ausführungsform liegt die Temperatur **T₂** von **H₂** unter der Temperatur **T₁** von **H₁**. Bevorzugt gilt dann, dass **T₂** > 0 °C, und der Quotient aus Temperatur **T₂**/**T₁** (°C/°C) im Bereich 0.1 bis 0.999, bevorzugter 0.2 bis 0.995, bevorzugter 0.3 bis 0.990 liegt.

Das Kondensat **K₁** kann sich neben dem aus der Kondensation von **H₁** erhaltenen Kondensat aus weiteren Heizdampfkondensaten zusammensetzen, etwa wenn im erfindungsgemäßen Verfahren verschiedene Heizdämpfe eingesetzt werden und damit auf verschiedene Komponenten **Q₁** von jeweils unterschiedlichen Heizdämpfen Energie übertragen wird. In dieser Ausgestaltung ergeben sich dann mehrere Kondensate **K₁**, die vereint werden können und im Schritt (f) zum Heizdampf **H₂** entspannt werden.

Eine solche bevorzugte Ausgestaltung ist beispielsweise in **Abbildung 3** gezeigt. Das im Kondensatbehälter <86> gesammelte Kondensat **K₁** ist eine Mischung der Kondensate <71> und <81> und zusätzlich <88>. Das Kondensat <81> ergibt sich aus der Kondensation des Heizdampfes <84> bei der Energieübertragung auf **G_{A}** im Zwischenverdampfer **VZ_{A}** <W9>. Das Kondensat <71> ergibt sich aus der Kondensation des Heizdampfes <74> bei der Energieübertragung auf **G_{A}** im Sumpfverdampfer **VS_{A}** <W8>.

Die Heißkondensate aus diesen beiden Verdampfern werden dann entsprechend den obigen Ausführungen zu einem Kondensatbehälter <86> geleitet. Der dort anfallende Heizdampf **H₂** <82> wird dann im regelbaren Dampfstrahler <89> verwendet, dessen Mischdampf als neuer Heizdampf **H₄** <84> wieder im Zwischenverdampfer **VZ_{A}** <W9> eingesetzt wird. Der Vorteil dieser Variante ist, dass das anfallende Heißkondensat weiter entspannt werden kann, um eine größere Niederdruckdampfmenge bereitstellen zu können.

**Abbildung 4** zeigt eine entsprechende Führung der Kondensat- und Heizdampfströme an der Rektifikationskolonne **RD_{X}** <3>.

### 4.8 Schritt (g) des erfindungsgemäßen Verfahrens

Im Schritt (g) des erfindungsgemäßen Verfahrens wird **H₂** mit weiterem Heizdampf **H₃** mit Druck **p₃** und Temperatur **T₃** gemischt, so dass ein Mischheizdampf **H₄** mit Druck **p₄** und Temperatur **T₄** erhalten wird, wobei **p₂** < **p₄** < **p₃** und insbesondere **p₁** < **p₃**.

Das Kondensat **K₁** und somit der Heizdampf **H₂** enthält noch Energie, die in keinem bekannten Verfahren zur Herstellung von Alkalimetallalkoholaten ausgenutzt wird. Aus energetischer und wirtschaftlicher Sicht ist das jedoch nicht sinnvoll. Diese Energie kann im Rahmen der vorliegenden Erfindung genutzt werden.

Die Mischung von **H₂** mit weiterem Heizdampf **H₃** kann mit jedem dem Fachmann bekannten Verfahren erfolgen, insbesondere durch Vereinigung der beiden Heizdämpfe per Venturidüse.

In einer bevorzugten Ausführungsform des erfindungsgemäßen Verfahrens wird der Dampf **H₂** mit weiterem Dampf, der einen Druck **p** < **p₃** hat, gemischt, und der dann erhaltene Dampf mit **H₃** gemischt.

Bevorzugt wird die Mischung von **H₂** mit weiterem Heizdampf **H₃** mittels eines, vorzugsweise regelbaren, Dampfstrahlers **DS** (Thermokompressors) durchgeführt. Der Thermokompressor wird dann sowohl mit weiterem Heizdampf **H₃**, der beispielsweise aus einem entsprechenden Dampfnetz stammt, als auch mit dem Niederdruckdampf **H₂** aus dem Kondensatbehälter gespeist, wodurch ein Mischdampf **H₄** entsteht, der dementsprechend das Wärmeträgermedium ist. Der Mischdampf ist in dieser Ausgestaltung demnach der Heizdampf **H₄**.

Ein solcher Dampfstrahler **DS** ist in **Abbildung 5** gezeigt. Er ist so ausgestaltet, dass er mit einem Treibdampf einer höheren Druckstufe betrieben wird und einen Saugdampf mit niedrigeren Druckstufe ansaugen kann, wodurch dann der Mischdampf entsteht, der als Wärmeträgermedium eingesetzt wird. Der Treibdampf ist im vorliegenden Fall der Heizdampf bzw. der Treibdampf **H₃**, mit dem der Heizdampf **H₂** als Saugdampf aus dem Kondensatbehälter angesaugt und mit dem Treibdampf **H₃** vermischt wird.

Der Vorteil einer solchen Ausgestaltung ist offensichtlich. Ein Teil der Energie des im Kondensatbehälter anfallenden Niederdruckdampfes kann genutzt und damit Energie und Kosten gespart werden. Eine solche Verfahrensweise kann auch aus einem anderen Grund vorteilhaft sein. Der eingesetzte Dampfstrahler kann dahingehend regelbar sein, dass die Mengen von Mitteldruckdampf **H₃** und Niederdruckdampf **H₂**, beispielsweise in Abhängigkeit von bestimmten Prozessparametern, eingestellt werden können. Die Saugdampfmenge stellt sich dabei über die Treibdampfmenge ein. Die Mengen von Mitteldruckdampf **H₃** und Niederdruckdampf **H₂** können beispielsweise in Abhängigkeit der Temperatur der mindestens einen Komponente **Q₁** eingestellt werden.

Der Druck **p₃** des Heizdampfes **H₃** ist größer als **p₂** und kann ansonsten in einem weiten Bereich gewählt werden. Die Erfindung ist gerade geeignet für Situationen, in denen Heizdampf **H₃** anfällt, auf dessen Druck und Temperatur man wenig Einfluss hat und der dann auf einen gegebenen Wert **H₄** eingestellt werden soll. Somit gilt **p₂** < **p₄** < **p₃**.

In einer besonderen Ausführungsform des erfindungsgemäßen Verfahrens ist, insbesondere dann, wenn **Q₁** und **Q₂** gleich sind, gilt außerdem **p₂** < **p₁** < **p₃**.

Der eingesetzte Heizdampf **H₃** hat bevorzugt einen Druck **p₃** im Bereich von 2 bar bis 80 bar, bevorzugt eine Temperatur **T₃** von 120 °C bis 300 °C, wobei gleichzeitig **p₄** < **p₃** und bevorzugt auch **p₁** < **p₃** sind.

### 4.9 Schritt (h) des erfindungsgemäßen Verfahrens

Im Schritt (h) wird Energie von Heizdampf **H₄** mit Druck **p₄** und Temperatur **T₄** auf mindestens eine Komponente **Q₂** übertragen.

Die Bedeutung der Komponente **Q₂** ist oben im Kontext des Schrittes (e), Abschnitt 4.6 definiert.

In einer bevorzugten Ausführungsform der vorliegenden Erfindung kondensiert insbesondere der Heizdampf **H₄** mindestens teilweise und es wird so ein Kondensat **K₂** erhalten wird.

**H₄** weist insbesondere eine Temperatur **T₄** von 101 °C bis 270 °C, vorzugsweise von 105 °C bis 250 °C, bevorzugt 110 °C bis 200 °C auf. **H₄** weist vorzugsweise einen Druck von 1 bis 55 bar, bevorzugt von 2 bis 5 bar, bevorzugter 4 bar auf. Als Heizdampf **H₄** kann auch ein Dampf mit einem Druck von > 30 bar eingesetzt werden.

Der Heizdampf **H₄** kondensiert insbesondere bei der Übertragung der Energie mindestens teilweise, wodurch vorzugsweise ein Kondensat **K₂** bei einem Druck von 1 bis 55 bar, vorzugsweise 2 bis 5 bar, besonders bevorzugt 4 bar und einer Temperatur von 101 °C bis 270 °C, vorzugsweise von 105 °C bis 250 °C, bevorzugt 110 °C bis 200 °C anfällt.

Das Heizdampfkondensat **K₂** wird bevorzugt in einem Kondensatbehälter gesammelt. Dazu können sämtliche dem Fachmann bekannten Kondensatbehälter genutzt werden, z.B. aus Stahl gefertigte Behälter.

Der Vorteil der vorliegenden Erfindung besteht unter anderem darin, dass die Abfolge der Schritte (f), (g), (h) im Kreislauf ausgeführt werden kann, und der im Anschluss an Schritt (h) durch die mindestens teilweise Kondensation von **H₄** anfallende Kondensat **K₂** wieder als Kondensat **K₁** in einer neuen Runde aus Schritt (f), (g), (h) eingesetzt wird.

Dies ist vor allem dann vorteilhaft, wenn **Q₁** und **Q₂** gleich sind.

In einer bevorzugten Ausführungsform der vorliegenden Erfindung wird deshalb der Heizdampf **H₄** mindestens teilweise kondensiert und so ein Kondensat **K₂** erhalten, mit welchem im Anschluss an Schritt (h) ein weiterer Schritt (f) durchgeführt wird, in dem das Kondensat **K₂** dann als Kondensat **K₁** eingesetzt wird.

Dies erlaubt es, das erfindungsgemäße Verfahren kontinuierlich durchzuführen und die Beheizung der entsprechenden Komponente **Q₁** und **Q₂** im Kreislauf durchzuführen.

Im erfindungsgemäßen Verfahren gleichen sich bevorzugt **p₁** und **p₄**, d.h. das Verhältnis aus **p₁**/**p₄** liegt bevorzugt im Bereich 0.5 bis 1.5, bevorzugter 0.8 bis 1.2, bevorzugter 0.9 bis 1.1, noch bevorzugter 0.99 bis 1.01, am bevorzugtesten 1.

### 5. Beispiele

### 5.1 Beispiel (nicht erfindungsgemäß)

Das Vergleichsbeispiel 1 wird in einer Apparatur entsprechend Abbildung 1 durchgeführt.

Ein Strom wässriger NaOH **S_{AE2}** <1A2> (48.5 Gew.-% NaOH, 51.5 Gew.-% Wasser) von 459 kg/h wird bei Raumtemperatur am Kopf einer Reaktionskolonne **RR_{A}** <1A> mit Kopfdruck von 1.6 bar abs. zugeführt.

Im Gegenstrom wird ein dampfförmiger Methanolstrom **S_{AE1}** <1A1>, der geringe Wasserverunreinigungen aufweist, von 6962 kg/h über dem Sumpf der Reaktionskolonne <1A> zugeführt.

Am Kopf der Reaktionskolonne wird ein Methanol-Wasser-Gemisch **S_{AB}** <1A5> erhalten, von dem 551 kg/h über einen Kondensator kondensiert und auf die Kolonne <1A> rückgeführt werden (nicht in den Abbildungen gezeigt), und 6421 kg/h weiter über einen Verdichter **VD₃₁** <11> zur Kolonne **RD_{A}** <2> geleitet werden.

Am Sumpf der Kolonne **RR_{A}** <1A> wird ein nahezu wasserfreier Produktstrom **S_{AS}** <1A4> aus der Kolonne geführt. Von diesem werden 1000 kg/h aus dem Prozess als Produktstrom geführt. Der andere Teil wird zum Sumpfverdampfer V_{SRRA} <WA1> geführt. 294 kg/h Heizdampf (11 bar abs., 190°C) werden über ein Regelventil <85> auf 4 bar abs. entspannt und damit über den Sumpfverdampfer **VS_{RRA}** <WA1 > Energie auf diesen Teil des entnommen Stroms **S_{AS}** <1A4> übertragen, wobei der Heizdampf kondensiert und wodurch der entnommene Produktstrom **S_{AS}**

<1A4> teilweise verdampft und so der gewünschte MeOH-Gehalt im Sumpfprodukt **S_{AS}** <1A4> eingestellt wird.

Der nicht kondensierte Teil des Kopfstroms **S_{AB}** <1A5> (6421 kg/h MeOH/Wasser Gemisch) wird über Verdichter **VD₃₁** <11> (Austrittsdruck 2,25 bar abs.) in die Rektifikationskolonne **RD_{A}** <2> geführt (Kopfdruck von Kolonne **RD_{A}** <2> = 2.2 bar abs.).

Am Kopf der Rektifikationskolonne **RD_{A}** <2> wird ein gegenüber dem Zulauf vom Verdichter **VD₃₁** <11> an Methanol angereichertes Methanol-Wasser-Gemisch **S_{DAB}** <24> erhalten, zusätzlich werden 878 kg/h frisches Methanol <25> am Kopf der Kolonne auf die Kolonne **RD_{A}** <2> gefahren. Am Sumpf der Rektifikationskolonne **RD_{A}** <2> erhält man einen gegenüber dem Kolonnenzulauf vom Verdichter mit Wasser angereicherten Strom **S_{DAS}** <22>. Ein Teil dieses Stroms wird über den Sumpfverdampfer **VS_{A}** <W8> in die Kolonne <2> rückgeführt, wobei der Rest 337 kg/h als Sumpfproduktstrom abgeführt wird.

Für den Betrieb des Sumpfverdampfers **VS_{A}** <W8> werden 3256 kg/h Heizdampf (11 bar abs., 190°C) über ein Regelventil <85> auf 4 bar abs. entspannt und über den Sumpfverdampfer **VS_{A}** <W8> Energie auf den rückgeführten Strom übertragen, wobei der Heizdampf kondensiert.

Die Heizdampfkondensatströme aus <WA1> und <W8> werden in einem Kondensatbehälter <86> zusammengeführt. Zur Rückführung in das Kondensatnetz wird der austretenden Kondensatstrom <87> von ca. 143°C mit Rückkühlwasser aus 75°C gekühlt. Dabei werden etwa 285 kW an das Rückkühlwasser übertragen.

### 5.2 Beispiel 2 (erfindungsgemäß)

Das Beispiel 2 wird in einer Apparatur entsprechend Abbildung 2 durchgeführt.

Es handelt sich um eine Wiederholung des Beispiels 1 mit folgenden Unterschieden:
Das gesammelte Kondensat im Kondensatbehälter <86> wird auf 1.5 bar abs. entspannt. Dabei verdampfen ca. 217 kg/h Kondensat <82>. Der Heizdampf <83> wird nicht über ein Regelventil <85> entspannt, sondern als Treibdampf für einen regelbaren Dampfstrahler <89> eingesetzt, um den Strom <82> einzusaugen und mit <83> zu vermischen. Am Austritt von <89> erhält man den Heizdampf <84> bei 4 bar abs. Dadurch dass zusätzlicher Heizdampf aus der Entspannung des Kondensats genutzt wird, werden nur 3048 kg/h frischer Heizdampf <83> für den Betrieb des Sumpfverdampfers VSA <W8> benötigt.

Es ergibt sich ein Strom <84> (rund 3265 kg/h), welcher bei Betrieb des Sumpfverdampfers **V_{SA}** <W8> als Kondensat <81> kondensiert. Der resultierende Kondensatstrom <81> wird dann wieder im Kondensatbehälter <86> eingeleitet.

Der Kondensatstrom <87> fällt nur mit einer Temperatur von ca. 111 °C an. Somit wird zur Kühlung auf 75 °C weniger Rückkühlwasser als in Beispiel 1 benötigt, und es werden nur rund 142 kW an das Kühlmedium übertragen, d.h. nur 50 % der nach Beispiel 1 nötigen Menge.

In Summe werden bei der Vorgehensweise nach Beispiel 2 nur 3342 kg/h Heizdampf (11 bar abs, 190°C) als Heizdampf für <WA1> und <WA8> benötigt. Gegenüber Beispiel 1 können so rund 6 % des 11 bar abs. Heizdampfes eingespart werden.

## Patentansprüche

1. Verfahren zur Herstellung mindestens eines Alkalimetallalkoholats der Formel M_{A}OR, wobei R ein C₁ bis C₆-Kohlenwasserstoffrest ist, und wobei M_{A} ausgewählt aus Natrium, Kalium, ist, wobei:
(a1) ein Eduktstrom **S_{AE1}** umfassend ROH mit einem Eduktstrom **S_{AE2}** umfassend M_{A}OH im Gegenstrom in einer Reaktivrektifikationskolonne **RR_{A}** zu einem Rohproduktgemisch **RP_{A}** umfassend M_{A}OR, Wasser, ROH, M_{A}OH umgesetzt wird,
wobei am unteren Ende von **RR_{A}** ein Sumpfproduktstrom **S_{AS}** umfassend ROH und M_{A}OR entnommen wird und am oberen Ende von **RR_{A}** ein Brüdenstrom **S_{AB}** umfassend Wasser und ROH entnommen wird,
(a2) und gegebenenfalls, gleichzeitig mit und räumlich getrennt von Schritt (a1), ein Eduktstrom **S_{BE1}** umfassend ROH mit einem Eduktstrom **S_{BE2}** umfassend M_{B}OH im Gegenstrom in einer Reaktivrektifikationskolonne **RR_{B}** zu einem Rohproduktgemisch **RP_{B}** umfassend M_{B}OR, Wasser, ROH, M_{B}OH umgesetzt wird, wobei M_{B} ausgewählt aus Natrium, Kalium ist,
wobei am unteren Ende von **RR_{B}** ein Sumpfproduktstrom **S_{BS}** umfassend ROH und M_{B}OR entnommen wird und am oberen Ende von **RR_{B}** ein Brüdenstrom **S_{BB}** umfassend Wasser und ROH entnommen wird,
(b) der Brüdenstrom **S_{AB}**, und, wenn Schritt (a2) durchgeführt wird, der Brüdenstrom **S_{BB}**, vermischt mit **S_{AB}** oder getrennt von **S_{AB}**, in eine Rektifikationskolonne **RD_{A}** geleitet wird, so dass in **RD_{A}** ein Gemisch **G_{A}** umfassend Wasser und ROH vorliegt,
(c) und **G_{A}** in **RD_{A}** in einen Brüdenstrom **S_{DAB}** umfassend ROH am oberen Ende von **RD_{A}** und einen Sumpfstrom **S_{DAS}** umfassend Wasser und ROH am unteren Ende von **RD_{A}** aufgetrennt wird,
wobei der Sumpfstrom **S_{DAS}** an einer Entnahmestelle **E_{AS}** am unteren Ende von **RD_{A}** aus **RD_{A}** geleitet wird und der Brüdenstrom **S_{DAB}** an einer Entnahmestelle **E_{AK}** am oberen Ende von **RD_{A}** aus **RD_{A}** geleitet wird,
(d) und gegebenenfalls **S_{DAS}**, ganz oder teilweise, in mindestens einer, von **RD_{A}** verschiedenen, Rektifikationskolonne **RD_{X},** in der dann ein Gemisch **G_{X}** umfassend Wasser und ROH vorliegt, in einen Brüdenstrom **S_{XB}** umfassend ROH am oberen Ende von **RD_{X}** und einen Sumpfstrom **S_{XS}** umfassend Wasser und gegebenenfalls ROH am unteren Ende von **RD_{X}** aufgetrennt wird, wobei der Sumpfstrom **S_{XS}** an einer Entnahmestelle **E_{XS}** am unteren Ende der mindestens einen Rektifikationskolonne **RD_{X}** aus dieser geleitet wird und der Brüdenstrom **S_{XB}** an einer Entnahmestelle **E_{XK}** am oberen Ende der mindestens einen Rektifikationskolonne **RD_{X}** aus dieser geleitet wird,
(e) Energie von einem Heizdampf **H₁** mit Druck **p₁** auf mindestens eine Komponente **Q₁** übertragen wird, wodurch der Heizdampf **H₁** mindestens teilweise kondensiert und so ein Kondensat **K₁** erhalten wird,
**dadurch gekennzeichnet, dass**
(f) **K₁** mindestens teilweise zu einem Heizdampf **H₂** mit Druck **p₂** entspannt wird, wobei **p₂ < p₁,**
(g) **H₂** mit weiterem Heizdampf **H₃** mit Druck **p₃** gemischt wird, so dass ein Heizdampf **H₄** mit Druck **p₄** erhalten wird, wobei **p₂ < p₄ < p₃,**
(h) Energie von **H₄** auf mindestens eine Komponente **Q₂** übertragen wird,
wobei **Q₁** und **Q₂** gleich oder verschieden voneinander sind,
und wobei **Q₁** und **Q₂** aus der Gruppe bestehend aus **S_{AE1}, S_{AE2}, RP_{A}, S_{AS}, S_{AB}, G_{A}, S_{DAS}, S_{DAB}** ausgewählt sind,
und wobei **Q₁** und **Q₂,** wenn Schritt (a2) durchgeführt wird, alternativ oder zusätzlich aus der Gruppe bestehend aus **S_{BE1}, S_{BE2}, RP_{B}, S_{BS}, S_{BB}** ausgewählt werden können,
und wobei **Q₁** und **Q₂,** wenn Schritt (d) durchgeführt wird, alternativ oder zusätzlich aus der Gruppe bestehend aus **G_{X}, S_{XB}, S_{XS}** ausgewählt werden können.

2. Verfahren nach Anspruch 1, wobei der Heizdampf **H₁** mit der mindestens einen Komponente **Q₁** kontaktiert wird, so dass Energie von **H₁** auf die mindestens eine Komponente **Q₁** übertragen wird und der Heizdampf **H₄** mit der mindestens einen Komponente **Q₂** kontaktiert wird, so dass Energie von **H₄** auf die mindestens eine Komponente **Q₂** übertragen wird.

3. Verfahren nach Anspruch 1, wobei Energie von Heizdampf **H₁** auf einen Wärmeträger **W₁** übertragen wird, und im Anschluss daran die Energie von **W₁** auf die mindestens eine Komponente **Q₁** übertragen wird und Energie von Heizdampf **H₄** auf **W₁** übertragen wird, und im Anschluss daran Energie von **W₁** auf die mindestens eine Komponente **Q₂** übertragen wird, wobei es sich bei **W₁** um keinen von **S_{AE1}, S_{AE2}, RP_{A}, S_{AS}, S_{AB}, G_{A}, S_{DAS}, S_{DAB}, S_{BE1}, S_{BE2}, RP_{B}, S_{BS}, S_{BB}, G_{X}, S_{XB}, S_{XS}** handelt.

4. Verfahren nach Anspruch 3, wobei **W₁** im Wesentlichen Wasser und/oder Alkohol ROH umfasst.

5. Verfahren nach Anspruch 4, wobei die Energieübertragung von **W₁** auf die mindestens eine Komponente **Q₁** bzw. **Q₂** erfolgt, in dem **W₁** mindestens teilweise mit **Q₁** bzw. **Q₂** gemischt wird, nachdem Energie von Heizdampf **H₁** bzw. **H₄ auf W₁** übertragen wurde.

6. Verfahren nach einem der Ansprüche 1 bis 5, wobei R ausgewählt ist aus Methyl, Ethyl.

7. Verfahren nach einem der Ansprüche 1 bis 6, wobei **p₁ < p₃.**

8. Verfahren nach einem der Ansprüche 1 bis 7, wobei **Q₁** und **Q₂** gleich sind.

9. Verfahren nach einem der Ansprüche 1 bis 8, wobei der Heizdampf **H₄** mindestens teilweise kondensiert und so ein Kondensat **K₂** erhalten wird, mit welchem im Anschluss an Schritt (h) ein weiterer Schritt (f) durchgeführt wird, in dem das Kondensat **K₂** dann als Kondensat **K₁** eingesetzt wird.

10. Verfahren nach einem der Ansprüche 1 bis 9, wobei das Verhältnis aus **p₁/p₄** im Bereich 0.5 bis 1.5 liegt.

11. Verfahren nach einem der Ansprüche 1 bis 10, wobei mindestens ein Teil des an der Entnahmestelle **E_{AK}** am oberen Ende der Rektifikationskolonne **RD_{A}** entnommenen Brüdenstroms **S_{AB}** der Reaktionskolonne **RR_{A}** mindestens als Teil des Eduktstroms **S_{AE1}** und, in den Fällen, in denen im erfindungsgemäßen Verfahren Schritt (a2) durchgeführt wird, alternativ oder zusätzlich der Reaktionskolonne **RR_{B}** mindestens als Teil des Eduktstroms **S_{BE1}** zugeführt wird.

12. Verfahren nach einem der Ansprüche 1 bis 11, wobei Schritt (d) durchgeführt wird.

13. Verfahren nach Anspruch 12, wobei mindestens ein Teil des an der Entnahmestelle **E_{XK}** am oberen Ende der mindestens einen Rektifikationskolonne **RD_{X}** entnommenen Brüdenstroms **S_{XB}** der Reaktionskolonne **RR_{A}** mindestens als Teil des Eduktstroms **S_{AE1}** und, in den Fällen, in denen im erfindungsgemäßen Verfahren Schritt (a2) durchgeführt wird, alternativ oder zusätzlich der Reaktionskolonne **RR_{B}** mindestens als Teil des Eduktstroms **S_{BE1}** zugeführt wird.

14. Verfahren nach einem der Ansprüche 1 bis 13, wobei Schritt (a2) durchgeführt wird.

15. Verfahren nach einem der Ansprüche 1 bis 14, welches kontinuierlich durchgeführt wird.

## Claims

1. Process for producing at least one alkali metal alkoxide of the formula M_{A}OR, where R is a C₁-C₆ hydrocarbon radical and M_{A} is selected from sodium, potassium, where:
(a1) a feed stream **S_{AE1}** comprising ROH is reacted with a feed stream **S_{AE2}** comprising M_{A}OH in countercurrent in a reactive rectification column **RR_{A}** to give a crude product mixture **RP_{A}** comprising M_{A}OR, water, ROH, M_{A}OH,
where a bottom product stream **S_{AS}** comprising ROH and M_{A}OR is taken off at the lower end of **RR_{A}** and a vapour stream **S_{AB}** comprising water and ROH is taken off at the upper end of **RR_{A},**
(a2) and optionally, simultaneously with and spatially separately from step (a1), a feed stream **S_{BE1}** comprising ROH is reacted with a feed stream **S_{BE2}** comprising M_{B}OH in countercurrent in a reactive rectification column **RR_{B}** to give a crude product mixture **RP_{B}** comprising M_{B}OR, water, ROH, M_{B}OH, where M_{B} is selected from sodium, potassium,
where a bottom product stream **S_{BS}** comprising ROH and M_{B}OR is taken off at the lower end of **RR_{B}** and a vapour stream **S_{BB}** comprising water and ROH is taken off at the upper end of **RR_{B},**
(b) the vapour stream **S_{AB},** and, when step (a2) is carried out, the vapour stream **S_{BB},** mixed with **S_{AB}** or separately from **S_{AB},** is introduced into a rectification column **RD_{A}** so that a mixture **G_{A}** comprising water and ROH is present in **RD_{A},**
(c) and **G_{A}** is separated in **RD_{A}** into a vapour stream **S_{DAB}** comprising ROH at the upper end of **RD_{A}** and a bottom stream **S_{DAS}** comprising water and ROH at the lower end of **RD_{A},**
where the bottom stream **S_{DAS}** is discharged from **RD_{A}** at an offtake point **E_{AS}** at the lower end of **RD_{A}** and the vapour stream **S_{DAS}** is discharged from **RD_{A}** at an offtake point **E_{AK}** at the upper end of **RD_{A},**
(d) and optionally **S_{DAS}** is, in its entirety or partly, separated in at least one rectification column **RD_{X}** which is different from **RD_{A}** and in which a mixture **Gx** comprising water and ROH is then present into a vapour stream **S_{XB}** comprising ROH at the upper end of **RD_{X}** and a bottom stream **Sxs** comprising water and optionally ROH at the lower end of **RD_{X},**
where the bottom stream **Sxs** is discharged from the at least one rectification column **RD_{X}** at an offtake point **E_{XS}** at the lower end of this column and the vapour stream **S_{XB}** is discharged from the at least one rectification column **RD_{X}** at an offtake point **E_{XK}** at the upper end of this column,
(e) energy is transferred from a heating vapour **H₁** having a pressure **p₁** to at least one component **Q₁,** as a result of which the heating vapour **H₁** condenses at least partially and a condensate **K₁** is thus obtained,
**characterized in that**
(f) **K₁** is at least partially depressurized to give a heating vapour **H₂** having a pressure **p₂,** where **p₂ < p₁,**
(g) **H₂** is mixed with further heating vapour **H₃** having a pressure **p₃** so as to give a heating vapour **H₄** having a pressure **p₄,** where **p₂ < p₄ < p₃,**
(h) energy is transferred from **H₄** to at least one component **Q₂,**
where **Q₁** and **Q₂** are identical or different from one another,
and where **Q₁** and **Q₂** are selected from the group consisting of **S_{AE1}, S_{AE2}, RP_{A}, S_{AS}, S_{AB}, G_{A}, S_{DAS}, S_{DAB},**
and where **Q₁** and **Q₂** can, when step (a2) is carried out, alternatively or additionally be selected from the group consisting of **S_{BE1}, S_{BE2}, RP_{B}, S_{BS}, S_{BB},**
and where **Q₁** and **Q₂** can, when step (d) is carried out, alternatively or additionally be selected from the group consisting of **G_{X}, S_{XB}, S_{XS}.**

2. Process according to Claim 1, wherein the heating vapour **H₁** is contacted with the at least one component **Q₁** so that energy is transferred from **H₁** to the at least one component **Q₁** and the heating vapour **H₄** is contacted with the at least one component **Q₂** so that energy is transferred from **H₄** to the at least one component **Q₂.**

3. Process according to Claim 1, wherein energy is transferred from heating vapour **H₁** to a heat transfer medium **W₁** and the energy of **W₁** is subsequently transferred to the at least one component **Q₁** and energy from heating vapour **H₄** is transferred to **W₁** and energy is subsequently transferred from **W₁** to the at least one component **Q₂,** where **W₁** is none of **S_{AE1}, S_{AE2}, RP_{A}, S_{AS}, S_{AB}, G_{A}, S_{DAS}, S_{DAB}, S_{BE1}, S_{BE2}, RP_{B}, S_{BS}, S_{BB}, G_{X}, S_{XB}, S_{XS}.**

4. Process according to Claim 3, wherein **W₁** comprises essentially water and/or alcohol ROH.

5. Process according to Claim 4, wherein the transfer of energy from **W₁** to the at least one component **Q₁** or **Q₂** occurs by **W₁** being mixed at least partly with **Q₁** or **Q₂** after energy has been transferred from heating vapour **H₁** or **H₄** to **W₁.**

6. Process according to any of Claims 1 to 5, wherein R is selected from methyl, ethyl.

7. Process according to any of Claims 1 to 6, wherein **p₁ < p₃.**

8. Process according to any of Claims 1 to 7, wherein **Q₁** and **Q₂** are identical.

9. Process according to any of Claims 1 to 8, wherein the heating vapour **H₄** is at least partially condensed so as to give a condensate **K₂** by means of which a further step (f) in which the condensate **K₂** is then used as condensate **K₁** is carried out after step (h).

10. Process according to any of Claims 1 to 9, wherein the ratio of **p₁/p₄** is in the range from 0.5 to 1.5.

11. Process according to any of Claims 1 to 10, wherein at least part of the vapour stream **S_{AB}** taken off at the offtake point **E_{AK}** at the upper end of the rectification column **RD_{A}** is fed to the reaction column **RR_{A}** at least as part of the feed stream **S_{AE1}** and, in cases in which step (a2) is carried out in the process of the invention, is alternatively or additionally fed to the reaction column **RR_{B}** at least as part of the feed stream **S_{BE1}.**

12. Process according to any of Claims 1 to 11, wherein step (d) is carried out.

13. Process according to Claim 12, wherein at least part of the vapour stream **S_{XB}** taken off at the offtake point **E_{XK}** at the upper end of the at least one rectification column **RD_{X}** is fed to the reaction column **RR_{A}** at least as part of the feed stream **S_{AE1}** and, in cases in which step (a2) is carried out in the process of the invention, is alternatively or additionally fed to the reaction column **RR_{B}** at least as part of the feed stream **S_{BE1}.**

14. Process according to any of Claims 1 to 13, wherein step (a2) is carried out.

15. Process according to any of Claims 1 to 14 which is carried out continuously.

## Revendications

1. Procédé de préparation d'au moins un alcoolate de métal alcalin de formule M_{A}OR, dans laquelle R représente un radical hydrocarboné en Ci-Ce et M_{A} est choisi parmi sodium, potassium, dans lequel :
(a1) un flux de départ S_{AE1} comprenant ROH est transformé avec un flux de départ S_{AE2} comprenant M_{A}OH à contre-courant dans une colonne de rectification réactive RR_{A} en un mélange produit brut RP_{A} comprenant M_{A}OR, de l'eau, ROH, M_{A}OH,
où, en l'extrémité inférieure de RR_{A}, est prélevé un flux de produit de fond S_{AS} comprenant ROH et M_{A}OR et, en l'extrémité supérieure de RR_{A}, est prélevé un flux de buées S_{AB} comprenant de l'eau et ROH,
(a2) et le cas échéant, simultanément avec l'étape (a1) et de manière spatialement séparée de celle-ci, un flux de départ S_{BE1} comprenant ROH est transformé avec un flux de départ S_{BE2} comprenant M_{B}OH à contre-courant dans une colonne de rectification réactive RR_{B} en un mélange produit brut RP_{B} comprenant M_{B}OR, de l'eau, ROH, M_{B}OH, M_{B} étant choisi parmi sodium, potassium,
où, en l'extrémité inférieure de RR_{B}, est prélevé un flux de produit de fond S_{BS} comprenant ROH et M_{B}OR et, en l'extrémité supérieure de RR_{B}, est prélevé un flux de buées S_{BB} comprenant de l'eau et ROH,
(b) le flux de buées S_{AB}, et, lorsque l'étape (a2) est mise en oeuvre, le flux de buées S_{BB}, mélangé avec S_{AB} ou séparément de S_{AB}, est/sont conduit(s) dans une colonne de rectification RD_{A} de telle sorte qu'un mélange G_{A} comprenant de l'eau et ROH se trouve dans RD_{A},
(c) et G_{A} dans RD_{A} est séparé en un flux de buées S_{DAB} comprenant ROH en l'extrémité supérieure de RD_{A} et en un flux de fond S_{DAS} comprenant de l'eau et ROH en l'extrémité inférieure de RD_{A},
le flux de fond S_{DAS} étant guidé hors de RD_{A} au niveau d'un site de prélèvement E_{AS} en l'extrémité inférieure de RD_{A} et le flux de buées S_{DAB} étant guidé hors de hors de RD_{A} au niveau d'un site de prélèvement E_{AK} en l'extrémité supérieure de RD_{A},
(d) et le cas échéant S_{DAS} est séparé, totalement ou partiellement, dans au moins une colonne de rectification RD_{X} différente de RD_{A}, dans laquelle se trouve alors un mélange Gx comprenant de l'eau et ROH, en un flux de buées S_{XB} comprenant ROH en l'extrémité supérieure de RD_{X} et en un flux de fond Sxs comprenant de l'eau et le cas échéant ROH en l'extrémité inférieure de RD_{X}, le flux de fond Sxs étant guidé hors de ladite au moins une colonne de rectification RD_{X} au niveau d'un site de prélèvement E_{XS} en l'extrémité inférieure de celle-ci et le flux de buées S_{XB} étant guidé hors de ladite au moins une colonne de rectification RD_{X} au niveau d'un site de prélèvement E_{XK} en l'extrémité supérieure de celle-ci,
(e) de l'énergie est transférée d'une vapeur chaude H₁ à la pression p₁ à au moins un composant Q₁, suite à quoi la vapeur chaude H₁ est condensée au moins partiellement et ainsi un condensat K₁ est obtenu,
**caractérisé en ce que**
(f) K₁ est détendu au moins partiellement en une vapeur chaude H₂ à la pression p₂, avec p₂ < p₁,
(g) H₂ est mélangée avec une autre vapeur chaude H₃ à la pression p₃, de telle sorte qu'une vapeur chaude H₄ à la pression p₄ est obtenue, avec p₂ < p₄ < p₃,
(h) de l'énergie de H₄ est transférée à au moins un composant Q₂,
Q₁ et Q₂ étant identiques ou différents l'un de l'autre, et Q₁ et Q₂ étant choisis dans le groupe constitué par S_{AE1}, S_{AE2}, RP_{A}, S_{AS}, S_{AB}, G_{A}, S_{DAS}, S_{DAB},
et Q₁ et Q₂, lorsque l'étape (a2) est mise en oeuvre, pouvant être choisis en variante ou en plus dans le groupe constitué par S_{BE1}, S_{BE2}, RP_{B}, S_{BS}, S_{BB},
et Q₁ et Q₂, lorsque l'étape (d) est mise en oeuvre, pouvant être choisis en variante ou en plus dans le groupe constitué par Gx, S_{XB}, Sxs.

2. Procédé selon la revendication 1, la vapeur chaude H₁ étant mise en contact avec ledit au moins un composant Q₁, de telle sorte que de l'énergie de H₁ est transférée audit au moins un composant Q₁ et la vapeur chaude H₄ étant mise en contact avec ledit au moins un composant Q₂ de telle sorte que de l'énergie de H₄ est transférée audit au moins un composant Q₂.

3. Procédé selon la revendication 1, de l'énergie de la vapeur chaude H₁ étant transférée à un caloporteur W₁ et l'énergie étant ensuite transférée de W₁ audit au moins un composant Q₁ et de l'énergie de la vapeur chaude H₄ étant transférée à W₁ et l'énergie étant ensuite transférée de W₁ audit au moins un composant Q₂, W₁ n'étant aucun des éléments S_{AE1}, S_{AE2}, RP_{A}, S_{AS}, S_{AB}, G_{A}, S_{DAS}, S_{DAB}, S_{BE1}, S_{BE2}, RP_{B}, S_{BS}, S_{BB}, Gx, S_{XB}, Sxs.

4. Procédé selon la revendication 3, W₁ comprenant essentiellement de l'eau et/ou de l'alcool ROH.

5. Procédé selon la revendication 4, le transfert d'énergie de W₁ audit au moins un composant Q₁ ou, selon le cas, Q₂ ayant lieu en ce que W₁ est mélangé au moins partiellement avec Q₁ ou, selon le cas, Q₂, après le transfert de l'énergie de la vapeur chaude H₁ ou, selon le cas, H₄ à W₁.

6. Procédé selon l'une des revendications 1 à 5, R étant choisi parmi méthyle, éthyle.

7. Procédé selon l'une des revendications 1 à 6, p₁ < p₃.

8. Procédé selon l'une des revendications 1 à 7, Q₁ et Q₂ étant identiques.

9. Procédé selon l'une des revendications 1 à 8, la vapeur chaude H₄ étant au moins partiellement condensée et ainsi un condensat K₂ étant obtenu, avec lequel une autre étape (f), dans laquelle le condensat K₂ est alors utilisé comme condensat K₁, est mise en oeuvre après l'étape (h).

10. Procédé selon l'une des revendications 1 à 9, le rapport de p₁/p₄ étant situé dans la plage de 0,5 à 1,5.

11. Procédé selon l'une des revendications 1 à 10, au moins une partie du flux du buées S_{AB}, prélevée au niveau du site de prélèvement E_{AK} en l'extrémité supérieure de la colonne de rectification RD_{A}, étant introduite dans la colonne de réaction RR_{A} au moins comme partie du flux de départ S_{AE1} et, dans les cas où, dans le procédé selon l'invention, l'étape (a2) est mise en oeuvre, en variante ou en plus dans la colonne de réaction RR_{B} au moins comme partie du flux de départ S_{BE1}.

12. Procédé selon l'une des revendications 1 à 11, l'étape (d) étant mise en oeuvre.

13. Procédé selon la revendication 12, au moins une partie du flux de buées S_{XB} prélevée au niveau du site de prélèvement E_{XK} en l'extrémité supérieure de ladite au moins une colonne de rectification R_{Dx} étant introduite dans la colonne de réaction RR_{A} au moins comme partie du flux de départ S_{AE1} et, dans les cas où, dans le procédé selon l'invention, l'étape (a2) est mise en oeuvre, en variante ou en plus dans la colonne de réaction RR_{B} au moins comme partie du flux de départ S_{BE1}.

14. Procédé selon l'une des revendications 1 à 13, l'étape (a2) étant mise en oeuvre.

15. Procédé selon l'une des revendications 1 à 14, qui est mis en oeuvre en continu.
